# EUROPEAN PATENT SPECIFICATION

(11) **EP 4 288 549 B1**
(45) Date of publication and mention of the grant of the patent: **03.06.2026**
(21) Application number: 22763678.4
(22) Date of filing: 05.03.2022
(51) Int. Cl.: A61K 35/76, A61K 48/00, A61P 7/00, C12N 9/64, C12N 15/67, C12N 15/86

(54) **CODON-OPTIMIZED NUCLEIC ACID ENCODING THE FIX PROTEIN**
CODONOPTIMIERTE NUKLEINSÄURE ZUR CODIERUNG DES FIX-PROTEINS
ACIDE NUCLÉIQUE À CODONS OPTIMISÉS CODANT POUR LA PROTÉINE DU FACTEUR FIX

(30) Priority: 05.03.2021 RU 2021105703
(43) Date of publication of application: 13.12.2023
(73) Proprietor: Joint Stock Company "Biocad", 198515 Saint Petersburg (RU)
(72) Inventor: PROKOFYEV, Alexander Vladimirovich, Saint Petersburg, 198206 (RU); GERSHOVICH, Pavel Mikhailovich, Saint Petersburg, 198328 (RU); STRELKOVA, Anna Nikolaevna, Yaransk, 612261 (RU); SPIRINA, Natalia Aleksandrovna, Saint Petersburg, 192283 (RU); SHUGAEVA, Tatiana Evgenievna, Moscow, 119021 (RU); MOROZOV, Dmitry Valentinovich, Saint Petersburg, 190000 (RU)
(74) Representative: Meissner Bolte Partnerschaft mbB
(86) International application number: PCT/RU2022/050073
(87) International publication number: WO 2022/186734

(56) References cited:
- WO-A1-2011/005968
- WO-A1-2016/004113
- WO-A1-2016/146757
- WO-A1-2016/210170
- WO-A1-2018/217731
- WO-A1-2020/084161
- WO-A2-2017/191274
- WO-A2-2020/118069
- RU-C2- 2 285 724

## Description

### FIELD OF THE INVENTION

The present application relates to the fields of genetics, gene therapy, and molecular biology. More specifically, the present invention relates to an isolated codon-optimized nucleic acid that encodes the FIX (coagulation factor IX) protein, an expression cassette and a vector based thereon, as well as an AAV5 (adeno-associated virus serotype 5)-based recombinant virus for increasing the expression of the FIX gene in target cells, and use thereof.

### BACKGROUND OF THE INVENTION

Gene therapy is one of the promising fields of modern medicine. It is mainly directed to the development of effective solutions for treating hereditary diseases, since only gene therapy methods make it possible to particularly influence the cause of these diseases. Among the large number of hereditary diseases, a group of pathologies associated with haemostasis disorders is most frequent.

Hemophilia is an X-linked disease associated with the absence or pronounced deficiency of plasma coagulation factors; it is characterized by coagulation disorders that are clinically manifested in the form of spontaneous or provokable, frequently uncontrolled, bleeding into joints, muscles and internal organs, etc.

Hemophilia B is caused by the absence or deficiency of plasma coagulation factor IX. In most cases, the disease has a family history, but sporadic mutations are identified in some cases. The vast majority of patients with hemophilia are males, there are identified cases of hemophilia in women, but they are extremely rare. Coagulation factor IX (FIX, Christmas factor) is a serine protease pro-enzyme which, in the presence of Ca2+ and membrane phospholipids, hydrolyzes the arginine-isoleucine bond in a factor X molecule to form activated factor X (FXa). The catalytic efficiency of factor IXa increases upon binding of cofactor, i.e. activated coagulation factor VIII (FVIIIa).

Factor IX is produced in the liver as inactive precursor protein which is processed in the endoplasmic reticulum and Golgi, where it undergoes multiple post-translational modifications of various types and is secreted into the bloodstream upon proteolytic cleavage of the propeptide. Factor IX, in the blood coagulation cascade, is activated after proteolytic cleavage by the activated factor XI (intrinsic pathway) or the activated factor VII (extrinsic pathway), with the formation of two polypeptide chains linked by a disulfide bond. Activated factor IX is slowly deactivated, typically by way of slow binding to antithrombin III, nexin-2, the protein Z-dependent protease inhibitor, and endocytic hepatocyte receptors, as well as degraded by neutrophil elastase.

Replacement therapy is currently used. After the development of the cryoprecipitation technique in 1966, the first coagulation factor product derived from donors' blood plasma was registered. In the 1980s, it was found that the plasma-derived coagulation factor products could be infected with viruses (HIV, hepatitis C), and it resulted in about 20,000 patients infected. This fact provided an impetus for developing methods for virus elimination and inactivation in the production of plasma products, and for creating novel non-plasma products. Into the production process of plasma-derived products (plasma derived - pdFIX), a heat treatment step was included; it made it possible to eliminate product infection. Simultaneously with the improvement of the process of producing plasma-derived products, studies were conducted on the development of coagulation factors using recombinant DNA technology. On the basis this technology, recombinant coagulation factor IX (rFIX) products were produced and registered in 1997. Recombinant DNA technology for producing therapeutic products makes it possible to significantly reduce the risk of viral contamination of the products. Currently, the replacement therapy therapeutic products for hemophilia are plasma-derived and recombinant products; however, they have a number of disadvantages.

The main problem in the production of plasma-derived products is the need for large volumes of plasma. Further, despite there have been no cases of infecting patients when using pdFIX since the late 1980s, manufacturers of these therapeutic products theoretically cannot exclude the possibility that they are virally infected.

The main disadvantages of FIX therapeutic products currently used for treating hemophilia include the following:
- theoretical possibility that patients are virally infected through plasma-derived products;
- high immunogenicity of plasma-derived and recombinant products;
- lower (as compared to plasma-derived products) efficacy of recombinant products;
- short blood circulation period of coagulation factors;
- need for frequent intravenous infusions (2-3 times a week);
- absence of widespread availability of lifelong replacement therapy.

The use of gene therapy therapeutic products for factor IX gene transduction is a fundamentally new and promising approach in comparison with existing therapy options: the gene therapy therapeutic product administered by intravenous infusion restores the production of the coagulation factor in the patient's body.

Delivery of the target gene into organism's cells using viral vectors, such as the AAV-based vector, is one of the main gene therapy methods.

Adeno-associated virus (AAV) is a small (25 nm), independent replication-defective, nonenveloped virus. Many different AAV serotypes have been described in human and primates. The adeno-associated virus genome is composed of(+ or -) single-stranded DNA (ssDNA) being about 4,700 nucleotides long. At the ends of a genomic DNA molecule there are accommodated terminal inverted repeats (ITRs). The genome comprises two open reading frames (ORFs), Rep and Cap, comprising several alternative reading frames encoding various protein products. The rep products are essential for AAV replication, whereas three capsid proteins (VP1, VP2, and VP3), along with other alternative products, are encoded by the Cap gene. VP1, VP2, and VP3 are present at 1:1:10 ratio to form an icosahedral capsid (Xie Q. et al. The atomic structure of adeno-associated virus (AAV-2), a vector for human gene therapy. Proc Natl Acad Sci USA, 2002; 99:10405-10410). During recombinant AAV (rAAV) vector production, an expression cassette flanked by ITR is packaged into an AAV capsid. The genes required for AAV replication are not included in the cassette. Recombinant AAV is considered to be one of the safest and most widely used viral vectors for *in vivo* gene transfer. Vectors can infect cells of multiple tissue types to provide strong and sustained transgene expression. They are also non-pathogenic, and have a low immunogenicity profile (High KA et al., "rAAV human trial experience" Methods Mol Biol. 2011; 807:429-57).

One of the urgent purposes of research in the area of development of effective gene therapy is codon optimization of genes of interest in vectors to achieve the maximum level of expression of the genes of interest, which, in turn, will allow using lower doses of the vector to achieve a significant effect.

One of the properties of the genetic code is degeneracy, i.e. the ability of different codons (trinucleotides) to encode the same amino acid. Such codons that are translated to the same amino acid are called synonymous codons. In natural sequences, one of the synonymous codons is selected randomly in the course of evolution, but the frequencies of usage of synonymous codons are different: each amino acid has more and less preferred ones. Codon optimization is a widely used technique to amplify the production of protein molecules, which provides a rational mapping of one of suitable synonymous codons to each amino acid in a protein sequence. One of the common principles of codon optimization involves the usage of the most frequent codons, whereas other approaches were introduced later, such as harmonization (reproduction of distribution of codon usage frequencies), but they do not always increase productivity. In addition to codon frequencies, the sequence GC content (ratio of guanine and cytosine to the total length of the sequence) may affect the production efficiency, in particular, it was shown that high GC content is associated with increased mRNA levels in mammalian cells (Grzegorz Kudla ET AL., High Guanine and Cytosine Content Increases mRNA Levels in Mammalian Cells, June 2006, Volume 4, Issue 6, e180, pp. 933-942). It is further worth noting that stable secondary structure elements of mRNA, i.e. those having a low free folding energy, may reduce the efficiency.

Different variants of codon-optimization of the sequence of a gene of interest may lead to the following (as compared to a wild-type gene):
a) expression levels of the genes of interest will be slightly increased;
b) expression levels of the genes of interest will be significantly increased;
c) expression levels of the genes of interest will remain approximately at the same level;
d) expression levels of the genes of interest will be lowered.

Thus, there is a need for producing a codon-optimized sequence of the FIX gene to increase the expression of the FIX gene in target cells and create a gene therapy therapeutic product based thereon.

### DESCRIPTION OF THE INVENTION

The authors of the present group of inventions found that the codon-optimized nucleic acid according to the invention, which encodes the FIX protein (coagulation factor IX), having the nucleotide sequence of SEQ ID NO: 2 (hFIXco-v1) or SEQ ID NO: 4 (hFIXco-v2 ), surprisingly showed increased level of FIX gene expression and increased level of production of coagulation factor IX protein by several times as compared to that of the wild-type gene encoding coagulation factor IX (hFIX-wt). These variants of the codon-optimized nucleic acid according to the invention having the nucleotide sequence of SEQ ID NO: 2 (hFIXco-v1) and SEQ ID NO: 4 (hFIXco-v2) are included into an expression cassette and vector based thereon, as well as into an AAV5 (adenoassociated virus serotype 5)-based recombinant virus.

### Brief description of the invention

In one aspect, the present invention relates to an isolated codon-optimized nucleic acid that encodes the FIX (coagulation factor IX) protein having the amino acid sequence of SEQ ID NO: 1, and that includes a nucleotide sequence that is selected from the group comprising: SEQ ID NO: 2 or SEQ ID NO: 4.

In one aspect, the present invention relates to an expression cassette that includes the above codon-optimized nucleic acid.

In some embodiments, the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments, the expression cassette includes a nucleotide sequence selected from the group comprising: SEQ ID NO: 3 or SEQ ID NO: 5.

In one aspect, the present invention relates to an expression vector that includes the above codon-optimized nucleic acid or any of the above expression cassettes.

In one aspect, the present invention relates to an isolated AAV5 (adeno-associated virus serotype 5)-based recombinant virus for increasing the FIX gene expression in target cells, comprising the above codon-optimized nucleic acid or any of the above expression cassettes.

In some embodiments, the AAV5-based recombinant virus has a capsid that includes the AAV5 protein VP1.

In some embodiments, the AAV5-based recombinant virus has a capsid that includes the AAV5 protein VP1 having the amino acid sequence of SEQ ID NO: 11.

In some embodiments, the AAV5-based recombinant virus has a capsid that includes the AAV5 protein VP1 having the amino acid sequence of SEQ ID NO: 11 with one or more point mutations.

In some embodiments, the AAV5-based recombinant virus has a capsid that includes the AAV5 protein VP1 having the amino acid sequence of SEQ ID NO: 14.

In some embodiments, the AAV5-based recombinant virus has a capsid that includes the AAV5 protein VP1 having the amino acid sequence of SEQ ID NO: 11 or the amino acid sequence of SEQ ID NO: 11 with one or more point mutations, and the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments, the AAV5-based recombinant virus has a capsid that includes the AAV5 protein VP1 having the amino acid sequence of SEQ ID NO: 11 or the amino acid sequence of SEQ ID NO: 11 with one or more point mutations, and the expression cassette comprises a nucleotide sequence selected from the group comprising SEQ ID NO: 3 or SEQ ID NO: 5.

In some embodiments, the AAV5-based recombinant virus has a capsid that includes the AAV5 protein VP1 having the amino acid sequence of SEQ ID NO: 11 with one or more point mutations, which is the amino acid sequence of SEQ ID NO: 14.

In one aspect, the present invention relates to a pharmaceutical composition for delivering the FIX gene to target cells, which includes any of the above AAV5-based recombinant viruses in combination with one or more pharmaceutically acceptable excipients.

In one aspect, the present invention relates to the use of any of the above AAV5-based recombinant viruses or the above composition to deliver the FIX gene to target cells.

In one aspect, the present invention relates to the use of any of the above AAV5-based recombinant viruses or the above composition for providing the FIX protein to a subject that has hemophilia B and/or that does not have fully functional copies of the FIX gene.

In one aspect, the present invention relates to the use of any of the above AAV5-based recombinant viruses or the above composition for treating hemophilia B in a subject that has hemophilia B.

In one aspect, the present invention relates to a method for providing the FIX protein to a subject having hemophilia B, said method comprising administering a therapeutically effective amount of any of the above AAV5-based recombinant viruses or the above composition into the cells of the subject in need thereof.

In one aspect, the present invention relates to a method for delivering the FIX gene to the target cells of a subject having hemophilia B, said method comprising administering any of the above AAV5-based recombinant viruses or the above composition into the cells of the subject.

In one aspect, the present invention relates to a method for treating hemophilia B in a subject, said method comprising administering a therapeutically effective amount of any of the above AAV5-based recombinant viruses or the above composition into a subject that has hemophilia B.

### Brief description of drawings

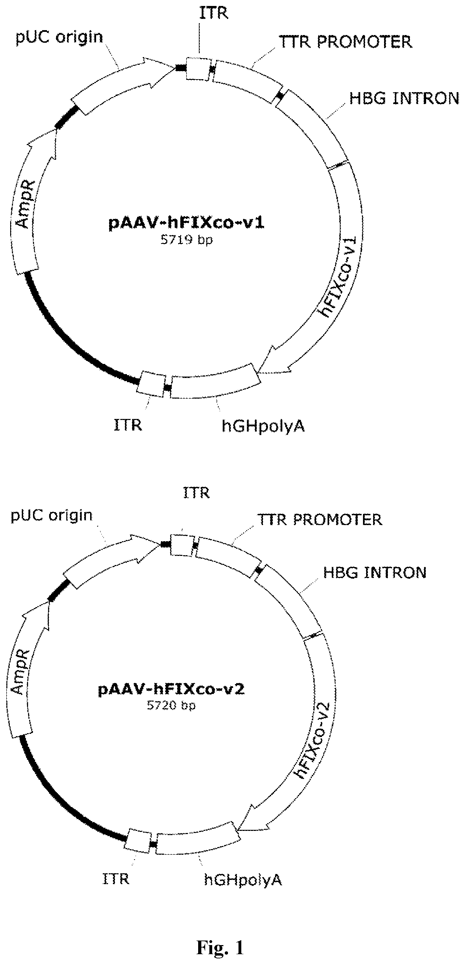

Figure 1 is a scheme of plasmids pAAV-hFIXco-v1and pAAV-hFIXco-v2 that are intended to produce AAV vectors with an expression cassette that comprises codon-optimized sequences of the human coagulation factor IX (FIX) gene hFIXco-v1 and hFIXco-v2, respectively, where
   hFIXco-v1 is a codon-optimized sequence of the human coagulation factor IX gene (variant No. 1);
   hFIXco-v2 is a codon-optimized sequence of the human coagulation factor IX gene (variant No. 2);
   AmpR is a beta-lactamase gene that provides resistance to ampicillin;
   pUC origin is a pUC replication origin in bacteria;
   ITR is inverted terminal repeats;
   TTR Promoter is a transthyretin gene promoter (transthyretin promoter);
   Poly A is a polyadenylation signal sequence, for increasing mRNA stability;
   HBG Intron is human beta globine intron.

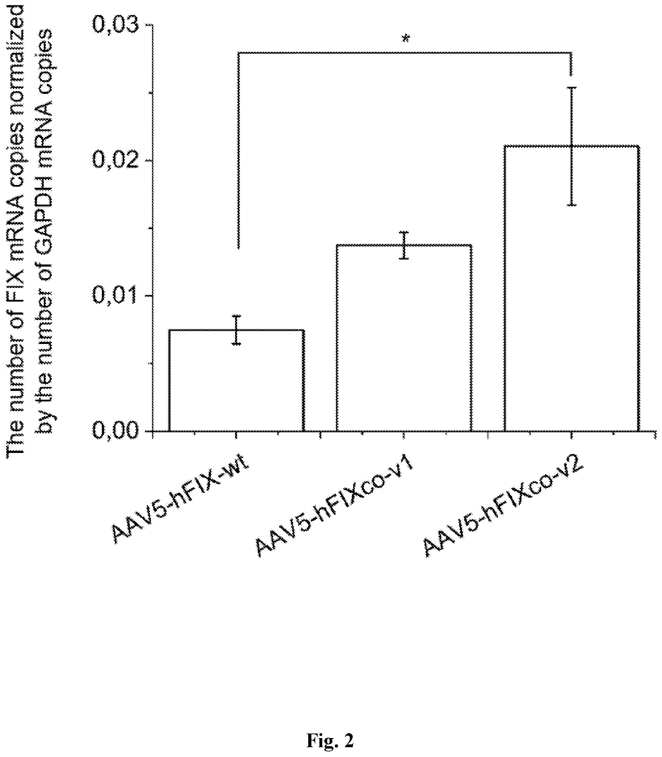

Figure 2 is a graph showing the level of expression of the FIX gene in Huh7 cells 7 days following transduction of the cells with AAV5-FIX viral product bearing the wild-type FIX gene (AAV5-hFIX-wt), with AAV5-FIX product bearing the codon-optimized gene hFIXco-v1 (AAV5-hFIXco-v1, and with AAV5-FIX product bearing the codon-optimized gene hFIXco-v2 (AAV5-hFIXco-v2) *(all variants of the FIX genes in AAV-FIX products contain the naturally-occurring Padua, R338L mutation).* * - p-value < 0.05. Statistical analysis was performed using two-way ANOVA with the Dunnett test.

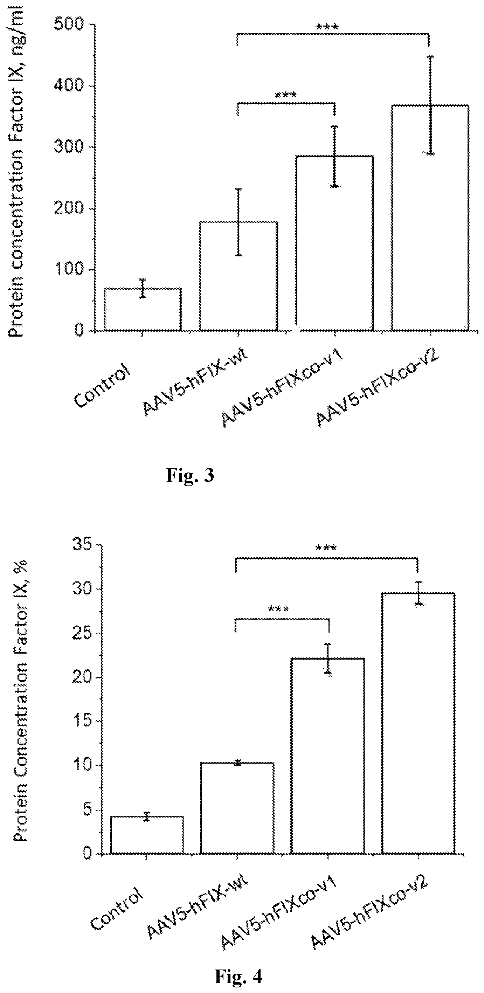

Figure 3 is a graph showing the concentration of the FIX protein in the culture fluid 7 days following transduction of Huh7 cells with AAV5-FIX viral product bearing the wild-type FIX gene (AAV5-hFIX-wt), with AAV5-FIX product bearing the codon-optimized gene hFIXco-v1 (AAV5-hFIXco-v1, and with AAV5-FIX product bearing the codon-optimized gene hFIXco-v2 (AAV5-hFIXco-v2) *(all variants of the FIX genes in AAV-FIX products contain the naturally-occurring Padua, R338L mutation).* Non-transduced Huh7 cells were used as control. *** - p-value < 0.001. Statistical analysis was performed using two-way ANOVA with the Dunnett test.
Figure 4 is a graph showing the activity of the FIX protein in the culture fluid 7 days following transduction of Huh7 cells with AAV5-FIX viral product bearing the wild-type FIX gene (AAV5-hFIX-wt), with AAV5-FIX product bearing the codon-optimized gene hFIXco-v1 (AAV5-hFIXco-v1, and with AAV5-FIX product bearing the codon-optimized gene hFIXco-v2 (AAV5-hFIXco-v2) *(all variants of the FIX genes in AAV-FIX products contain the naturally-occurring Padua, R338L mutation).* Non-transduced Huh7 cells were used as control. *** - p-value < 0.001. Statistical analysis was performed using two-way ANOVA with the Dunnett test.

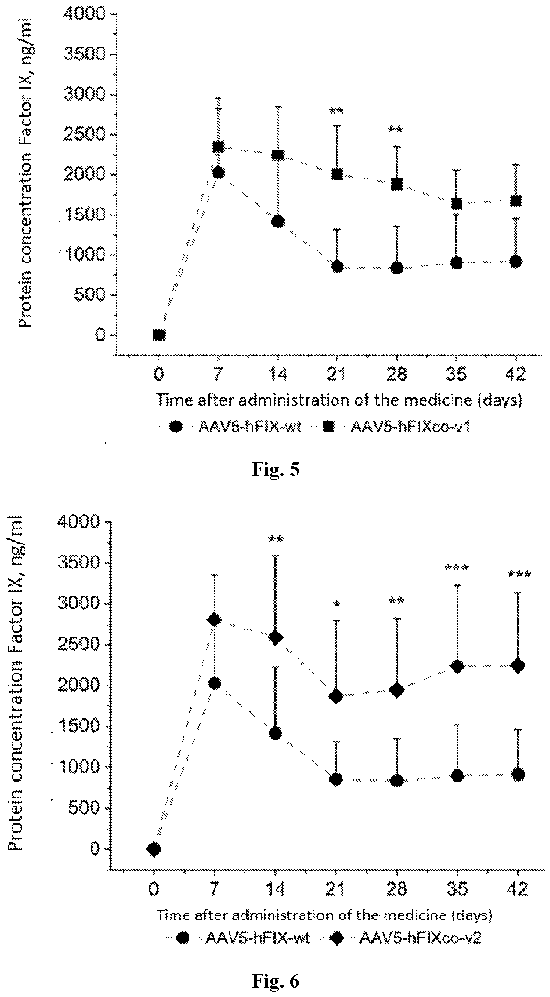

Figure 5 is a graph showing the content of the coagulation factor IX protein in the blood plasma of experimental animals following intravenous administration of the AAV5-FIX product bearing the wild-type FIX gene (AAV5-hFIX-wt) and AAV5-FIX product bearing the codon-optimized gene hFIXco-v1 (AAV5-hFIXco-v1) *(all versions of the FIX genes in the AAV-FIX products contain naturally-occurring Padua, R338L mutation).* Mean values ± standard deviation (n = 10). *** - p-value < 0.001; ** - p-value < 0.01; * - p-value < 0.05. Statistical analysis was performed using one-way ANOVA with the Dunnett test.
Figure 6 is a graph showing the content of the coagulation factor IX protein in the blood plasma of experimental animals following intravenous administration of the AAV5-FIX product bearing the wild-type FIX gene (AAV5-hFIX-wt) and AAV5-FIX product bearing the codon-optimized gene hFIXco-v2 (AAV5-hFIXco-v2) *(all versions of the FIX genes in the AAV-FIX products contain naturally-occurring Padua, R338L mutation).* Mean values ± standard deviation (n = 10). *** - p-value < 0.001; ** - p-value < 0.01; * - p-value < 0.05. Statistical analysis was performed using one-way ANOVA with the Dunnett test.

### Definitions and general methods

Unless defined otherwise herein, all technical and scientific terms used in connection with the present invention will have the same meaning as is commonly understood by those skilled in the art.

Furthermore, unless otherwise required by context, singular terms shall include plural terms, and the plural terms shall include the singular terms. Typically, the present classification and methods of cell culture, molecular biology, immunology, microbiology, genetics, analytical chemistry, organic synthesis chemistry, medical and pharmaceutical chemistry, as well as hybridization and chemistry of protein and nucleic acids described herein are well known by those skilled and widely used in the art. Enzyme reactions and purification methods are performed according to the manufacturer's guidelines, as is common in the art, or as described herein.

"Isolated" means altered or removed from the natural state. For example, a nucleic acid or a peptide naturally present in an animal is not "isolated", but the same nucleic acid or peptide partially or completely separated from the coexisting materials of its natural state is "isolated". An isolated nucleic acid or protein can exist substantially in purified form, or can exist in a non-native environment such as, for example, a genetically modified cell.

The terms "naturally occurring," "native," or "wild-type" are used to describe an object that can be found in nature as distinct from being artificially produced. For example, a protein or nucleotide sequence present in an organism (including a virus), which can be isolated from a source in nature and that has not been intentionally modified by a person in the laboratory, is naturally occurring.

The term "genome" refers to the complete genetic material of an organism.

As used in the present description and claims that follow, unless otherwise dictated by the context, the words "include" and "comprise," or variations thereof such as "having," "includes", "including", "comprises," or "comprising," will be understood to imply the inclusion of a stated integer or group of integers but not the exclusion of any other integer or group of integers.

### Protein (Peptide)

As used in the present description, the terms "peptide", "polypeptide" and "protein" are used interchangeably, and they refer to a compound consisting of amino acid residues that are covalently linked by peptide bonds. A protein or peptide must contain at least two amino acids, and no limitation is placed on the maximum number of amino acids that can comprise a protein's or peptide's sequence. Polypeptides include any peptide or protein comprising two or more amino acids joined to each other by peptide bonds. As used in the present description, the term refers to both short chains, which also commonly are referred to in the art, for example, as peptides, oligopeptides and oligomers, and to longer chains, which generally are referred to in the art as proteins, of which there are many types. "Polypeptides" include, inter alia, for example, biologically active fragments, substantially homologous polypeptides, oligopeptides, homodimers, heterodimers, variants of polypeptides, modified polypeptides, derivatives, analogs, fusion proteins. The polypeptides include natural peptides, recombinant peptides, synthetic peptides, or a combination thereof.

### Nucleic acid molecules

The terms "nucleic acid", "nucleic sequence", "nucleic acid sequence", "polynucleotide", "oligonucleotide", "polynucleotide sequence" and "nucleotide sequence", used interchangeably in the present description, mean a precise sequence of nucleotides, modified or not, determining a fragment or a region of a nucleic acid, containing unnatural nucleotides or not, and being either a double-strand DNA or RNA, a single-strand DNA or RNA, or transcription products of said DNAs.

As used in the present description, polynucleotides include, as non-limiting examples, all nucleic acid sequences which are obtained by any means available in the art, including, as non-limiting examples, recombinant means, i.e. the cloning of nucleic acid sequences from a recombinant library or a cell genome, using ordinary cloning technology and PCR and the like, and by synthetic means.

It should also be included here that the present invention does not relate to nucleotide sequences in their natural chromosomal environment, i.e. in a natural state. The sequences of the present invention have been isolated and/or purified, i.e., they were sampled directly or indirectly, for example by copying, their environment having been at least partially modified. Thus, isolated nucleic acids obtained by recombinant genetics, by means, for example, of host cells, or obtained by chemical synthesis should also be mentioned here.

Unless otherwise indicated, the term nucleotide sequence encompasses its complement. Thus, a nucleic acid having a particular sequence should be understood as one which encompasses the complementary strand thereof with the complementary sequence thereof.

### Adeno-associated virus (AAV)

Viruses of the Parvoviridae family are small DNA-containing animal viruses. The Parvoviridae family may be divided into two subfamilies: the Parvovirinae, which members infect vertebrates, and the Densovirinae, which members infect insects. By 2006, there have been 11 serotypes of adeno-associated virus described (Mori, S. ET AL., 2004, "Two novel adeno-associated viruses from cynomolgus monkey: pseudotyping characterization of capsid protein", Virology, T. 330 (2): 375-83). All of the known serotypes can infect cells from multiple tissue types. Tissue specificity is determined by the capsid protein serotype; therefore, the adeno-associated virus-based vectors are constructed by assigning the desired serotype. Further information on parvoviruses and other members of the Parvoviridae is described in the literature (Kenneth I. Berns, «Parvoviridae: The Viruses and Their Replication», Chapter 69 in Fields Virology (3d Ed. 1996)).

The genomic organization of all known AAV serotypes is very similar. The genome of AAV is a linear, single-stranded DNA molecule that is less than about 5000 nucleotides (nt) in length. Inverted terminal repeats (ITRs) flank the unique coding nucleotide sequences of replication of non-structural proteins (Rep) and structural proteins (Cap). The Cap gene encodes the VP proteins (VP1, VP2, and VP3) which form the capsid. The terminal 145 nucleotides are self-complementary and are organized such that an energetically stable intramolecular duplex forming a T-shaped hairpin may be formed. Such hairpin structures function as an origin for virus DNA replication, serving as primers for the cellular DNA polymerase complex. Following wild-type AAV (wtAAV) infection in mammalian cells, the Rep genes (e.g. Rep78 and Rep52) are expressed using the P5 promoter and the P19 promoter, respectively, and the both Rep proteins have a certain function in the replication of the viral genome. A splicing event in the Rep open reading frame (Rep ORF) results in the expression of actually four Rep proteins (e.g. Rep78, Rep68, Rep52, and Rep40). However, it has been shown that the unspliced mRNA encoding Rep78 and Rep52 proteins is sufficient for AAV vector production in mammalian cells.

### Vector

The term "vector" as used herein means a nucleic acid molecule capable of transporting another nucleic acid to which it has been linked. Furthermore, the term "vector" herein refers to a viral particle capable of transporting a nucleic acid.

As used in the present description, the term "expression" is defined as the transcription and/or translation of a particular nucleotide sequence driven by its promoter.

### Use

"Gene therapy" is the insertion of genes into subject's cells and/or tissues to treat a disease, typically hereditary diseases, in which a defective mutant allele is replaced with a functional one.

"Treat", "treating" and "treatment" refer to a method of alleviating or abrogating a biological disorder and/or at least one of its attendant symptoms. As used herein, to "alleviate" a disease, disorder or condition means reducing the severity and/or occurrence frequency of the symptoms of a disease, disorder, or condition. Further, references herein to "treatment" include references to curative, palliative and prophylactic treatment.

In one aspect, the subject of treatment, or patient, is a mammal, preferably a human subject. Said subject may be either male or female, of any age.

The term "disorder" means any condition that would benefit from treatment with the compound of the present invention.

"Disease" is a state of health of a subject where the subject cannot maintain homeostasis, and where if the disease is not ameliorated then the subject's health continues to deteriorate.

The terms "subject," "patient," "individual," and the like are used interchangeably in the present description, and they refer to any animal which is amenable to the methods described in the present description. In certain non-limiting embodiments, the subject, patient or individual is a human. Said subject may be either male or female, of any age.

"Therapeutically effective amount" or "effective amount" refers to that amount of the therapeutic agent being administered which will relieve to some extent one or more of the symptoms of the disease being treated.

### Detailed description of the invention

### Nucleic acid

In one aspect, the present invention relates to an isolated codon-optimized nucleic acid that encodes the FIX protein (coagulation factor IX) having the amino acid sequence 
MQRVNMIMAESPGLITICLLGYLLSAECTVFLDHENANKILNRPKRYNSGKLEEFVQGN
LERECMEEKCSFEEAREVFENTERTTEFWKQYVDGDQCESNPCLNGGSCKDDINSYEC
WCPFGFEGKNCELDVTCNIKNGRCEQFCKNSADNKVVCSCTEGYRLAENQKSCEPAVP
FPCGRVSVSQTSKLTRAETVFPDVDYVNSTEAETILDNITQSTQSFNDFTRVVGGEDAKP
GQFPWQVVLNGKVDAFCGGSIVNEKWIVTAAHCVETGVKITVVAGEHNIEETEHTEQK
RNVIRIIPHHNYNAAINKYNHDIALLELDEPLVLNSYVTPICIADKEYTNIFLKFGSGYVSG
 
WGRVFHKGRSALVLQYLRVPLVDRATCLLSTKFTIYNNMFCAGFHEGGRDSCQGDSGG
PHVTEVEGTSFLTGIISWGEECAMKGKYGIYTKVSRYVNWIKEKTKLT (SEQ ID NO:1),
 and that includes a nucleotide sequence selected from the group comprising: 
ATGCAGCGGGTCAACATGATCATGGCGGAGTCGCCGGGCCTGATCACGATCTGCCT
CCTCGGGTACCTGCTCTCCGCCGAGTGCACCGTGTTCCTGGACCACGAGAACGCCAA
CAAGATCCTCAACCGGCCCAAGCGCTACAACTCCGGCAAGCTGGAGGAGTTCGTGC
AGGGGAACCTCGAGCGCGAGTGCATGGAGGAGAAGTGCTCGTTCGAGGAGGCGCG
GGAGGTGTTCGAGAACACCGAGCGCACCACGGAGTTCTGGAAGCAGTACGTGGACG
GGGACCAGTGCGAGTCGAACCCGTGCCTCAACGGGGGTCGTGCAAGGACGACATC
AACTCGTACGAGTGCTGGTGCCCCTTCGGCTTCGAGGGCAAGAACTGCGAGCTGGA
CGTGACCTGCAACATCAAGAACGGGCGCTGCGAGCAGTTCTGCAAGAACAGCGCCG
ACAACAAGGTGGTCTGCTCCTGCACCGAGGGGTACCGCCTCGCGGAGAACCAGAAG
TCCTGCGAGCCGGCCGTGCCCTTCCCCTGCGGCCGCGTGTCCGTCAGCCAGACGTCG
AAGCTGACGCGCGCCGAGACCGTCTTCCCGGACGTGGACTACGTGAACTCGACGGA
GGCCGAGACCATCCTGGACAACATCACCCAGAGCACCCAGTCCTTCAACGACTTCA
CGCGGGTGGTCGGCGGCGAGGACGCCAAGCCCGGGCAGTTCCCGTGGCAGGTCGTC
CTCAACGGGAAGGTCGACGCGTTCTGCGGCGGGAGCATCGTGAACGAGAAGTGGAT
CGTGACCGCCGCGCACTGCGTCGAGACGGGCGTGAAGATCACCGTGGTGGCCGGGG
AGCACAACATCGAGGAGACGGAGCACACCGAGCAGAAGCGGAACGTGATCCGCAT
CATCCCGCACCACAACTACAACGCCGCCATCAACAAGTACAACCACGACATCGCGC
TCCTCGAGCTGGACGAGCCGCTGGTCCTCAACTCCTACGTCACGCCGATCTGCATCG
CCGACAAGGAGTACACGAACATCTTCCTGAAGTTCGGGAGCGGCTACGTCTCGGGC
TGGGGCCGCGTGTTCCACAAGGGGCGCAGCGCGCTCGTGCTCCAGTACCTGCGGGT
CCCCCTGGTCGACCGCGCGACCTGCCTCCTCTCCACGAAGTTCACGATCTACAACAA
CATGTTCTGCGCGGGGTTCCACGAGGGCGGCCGGGACAGCTGCCAGGGCGACAGCG
GGGGCCCGCACGTGACGGAGGTGGAGGGCACGAGCTTCCTGACCGGGATCATCTCG
TGGGGCGAGGAGTGCGCGATGAAGGGGAAGTACGGCATCTACACCAAGGTCAGCC
GGTACGTGAACTGGATCAAGGAGAAGACGAAGCTGACG (SEQ ID NO: 2)
 or

ATGCAGCGGGTGAACATGATCATGGCCGAGTCCCCAGGCCTGATTACCATCTGTCTG
CTGGGCTACCTGCTGAGCGCCGAATGCACCGTGTTCTGGACCACGAGAACGCCAA
CAAGATCCTGAACCGCCCTAAGCGGTACAACTCCGGCAAGCTGGAGGAGTTTGTGC
AGGGCAATCTGGAGCGGGAGTGTATGGAGGAGAAGTGCAGCTTCGAGGAGGCCAG
GGAGGTGTTCGAGAACACCGAGAGGACCACCGAGTTCTGGAAGCAGTATGTGGACG
GCGACCAGTGCGAGTCTAATCCTTGTCTGAATGGCGGGAGCTGCAAGGACGACATC

AACAGCTACGAGTGCTGGTGCCCTTTCGGCTTCGAGGGCAAGAATTGCGAGCTGGA
CGTGACCTGCAACATCAAGAACGGCCGGTGTGAGCAGTTCTGCAAGAACAGCGCCG
ACAACAAGGTGGTGTGCTCCTGCACCGAGGGATACAGGCTGGCCGAGAATCAGAAG
AGCTGTGAGCCCGCCGTGCCATTCCCCTGTGGCAGAGTGTCTGTGAGCCAGACCAGC
AAGCTGACCAGAGCCGAGACCGTGTTTCCAGACGTGGACTACGTGAACAGCACCGA
GGCCGAGACCATCCTGGATAATATCACCCAGTCCACCCAGAGCTTCAACGACTTCAC
CAGAGTGGTGGGAGGCGAGGATGCCAAGCCAGGACAGTTTCCCTGGCAGGTGGTGC
TGAATGGCAAGGTGGACGCCTTCTGCGGAGGCAGCATCGTGAACGAGAAGTGGATT
GTGACCGCAGCCCACTGCGTGGAGACTGGCGTGAAGATTACCGTGGTCGCCGGCGA
GCACAATATCGAAGAGACCGAGCACACCGAGCAGAAGCGCAACGTGATCCGGATC
ATCCCTCACCACAACTACAACGCAGCCATCAACAAGTACAACCACGACATCGCCCT
GCTGGAGCTGGACGAGCCACTGGTGCTGAACTCTTACGTGACCCCTATCTGCATCGC
CGACAAGGAGTACACCAACATCTTCCTGAAGTTCGGCAGCGGCTACGTGAGCGGAT
GGGGCAGAGTGTTTCACAAGGGCAGGAGCGCCCTGGTGCTGCAGTATCTGAGAGTG
CCACTGGTGGACAGAGCTACCTGCCTGCTGAGCACCAAGTTCACCATCTACAACAAC
ATGTTCTGCGCCGGCTTCCACGAGGGGGGAAGAGACTCTTGCCAGGGCGATTCCGG
CGGACCACACGTGACCGAAGTGGAGGGCACCAGCTTCCTGACCGGCATCATCTCCT
GGGGCGAGGAATGCGCCATGAAGGGCAAGTACGGCATCTACACCAAGGTGAGCAG
GTACGTGAACTGGATCAAGGAGAAGACCAAGCTGACC (SEQ ID NO: 4).

An "isolated" nucleic acid molecule is one which is identified and separated from at least one nucleic acid molecule-impurity, which the former is typically bound to in the natural source of nuclease nucleic acid. An isolated nucleic acid molecule is different from the form or set in which it is found under natural conditions. Thus, an isolated nucleic acid molecule is different from a nucleic acid molecule that exists in cells under natural conditions. An isolated nucleic acid molecule however includes a nucleic acid molecule located in cells in which the nuclease is normally expressed, for example, if the nucleic acid molecule has a chromosomal localization that is different from its localization in cells under natural conditions.

The above codon-optimized nucleic acid was obtained by codon-optimizing a wild-type nucleic acid having the nucleotide sequence

ATGCAGCGCGTGAACATGATCATGGCAGAATCACCAGGCCTCATCACCATCTGCCTT
TTAGGATATCTACTCAGTGCTGAATGTACAGTTTTCTTGATCATGAAAACGCCAAC
AAAATTCTGAATCGGCCAAAGAGGTATAATTCAGGTAAATTGGAAGAGTTTGTTCA
AGGGAACCTTGAGAGAGAATGTATGGAAGAAAAGTGTAGTTTTGAAGAAGCACGA
GAAGTTTTTGAAAACACTGAAAGAACAACTGAATTTTGGAAGCAGTATGTTGATGG
AGATCAGTGTGAGTCCAATCCATGTTTAAATGGCGGCAGTTGCAAGGATGACATTA

ATTCCTATGAATGTTGGTGTCCCTTTGGATTTGAAGGAAAGAACTGTGAATTAGATG
TAACATGTAACATTAAGAATGGCAGATGCGAGCAGTTTTGTAAAAATAGTGCTGAT
AACAAGGTGGTTTGCTCCTGTACTGAGGGATATCGACTTGCAGAAAACCAGAAGTC
CTGTGAACCAGCAGTGCCATTTCCATGTGGAAGAGTTTCTGTTTCACAAACTTCTAA
GCTCACCCGTGCTGAGACTGTTTTTCCTGATGTGGACTATGTAAATTCTACTGAAGCT
GAAACCATTTTGGATAACATCACTCAAAGCACCCAATCATTTAATGACTTCACTCGG
GTTGTTGGTGGAGAAGATGCCAAACCAGGTCAATTCCCTTGGCAGGTTGTTTTGAAT
GGTAAAGTTGATGCATTCTGTGGAGGCTCTATCGTTAATGAAAAATGGATTGTAACT
GCTGCCCACTGTGTTGAAACTGGTGTTAAAATTACAGTTGTCGCAGGTGAACATAAT
ATTGAGGAGACAGAACATACAGAGCAAAAGCGAAATGTGATTCGAATTATTCCTCA
CCACAACTACAATGCAGCTATTAATAAGTACAACCATGACATTGCCCTTCTGGAACT
GGACGAACCCTTAGTGCTAAACAGCTACGTTACACCTATTTGCATTGCTGACAAGGA
ATACACGAACATCTTCCTCAAATTTGGATCTGGCTATGTAAGTGGCTGGGGAAGAGT
CTTCCACAAAGGGAGATCAGCTTTAGTTCTTCAGTACCTTAGAGTTCCACTTGTTGA
CCGAGCCACATGTCTTCTATCTACAAAGTTCACCATCTATAACAACATGTTCTGTGCT
GGCTTCCATGAAGGAGGTAGAGATTCATGTCAAGGAGATAGTGGGGGACCCCATGT
TACTGAAGTGGAAGGGACCAGTTTCTTAACTGGAATTATTAGCTGGGGTGAAGAGT
GTGCAATGAAAGGCAAATATGGAATATATACCAAGGTATCCCGGTATGTCAACTGG
ATTAAGGAAAAAACAAAGCTCACT (SEQ ID NO: 17)

As a result of codon optimization of the FIX protein-encoding wild-type nucleic acid having the nucleotide sequence of SEQ ID NO: 17, a number of codon-optimized nucleic acids were obtained, which were further tested for the level of protein production as compared to the control (wild-type nucleic acid with SEQ ID NO: 17).

All codon-optimized nucleic acids showed increased level of FIX protein production as compared to that of the wild-type; further, the codon-optimized nucleic acid of the invention having the nucleotide sequence of SEQ ID NO: 2 (hFIXco-v1) and the codon-optimized nucleic acid of the invention having the nucleotide sequence of SEQ ID NO: 4 (hFIXco-v2) surprisingly showed the best results, in particular, increased level of FIX gene expression, several-fold increased level of FIX protein production as compared to those of the wild type (see examples 2, 3 and 4).

### Expression cassette. Expression vector.

In one aspect, the present invention relates to an expression cassette that includes the above codon-optimized nucleic acid that encodes the FIX protein (coagulation factor IX).

The term "cassette which expresses" or "expression cassette", as used herein, refers in particular to a DNA fragment that is capable, in an appropriate setting, of triggering the expression of a polynucleotide encoding a polypeptide of interest that is included in said expression cassette. When introduced into a host cell, the expression cassette is, inter alia, capable of engaging cellular mechanisms to transcribe the polynucleotide encoding the polypeptide of interest into RNA that is then typically further processed and eventually translated into the polypeptide of interest. The expression cassette may be contained in an expression vector.

The expression cassette of the present invention comprises a promoter as an element. The term "promoter" as used herein refers in particular to a DNA element that promotes the transcription of a polynucleotide to which the promoter is operably linked. The promoter may further form part of a promoter/enhancer element. Although the physical boundaries between the "promoter" and "enhancer" elements are not always clear, the term "promoter" typically refers to a site on the nucleic acid molecule to which an RNA polymerase and/or any associated factors binds and at which transcription is initiated. Enhancers potentiate promoter activity temporally as well as spatially. Many promoters are known in the art to be transcriptionally active in a wide range of cell types. Promoters can be divided into two classes, those that function constitutively and those that are regulated by induction or derepression. The both classes are suitable for protein expression. Promoters that are used for high-level production of polypeptides in eukaryotic cells and, in particular, in mammalian cells, should be strong and preferably active in a wide range of cell types. Strong constitutive promoters which are capable of driving expression in many cell types are well known in the art and, therefore, it is not herein necessary to describe them in detail. In accordance with the idea of the present invention, it is preferable to use the TTR promoter. The TTR promoter or TTR promoter/enhancer is particularly suitable as a promoter in the expression cassette of the present invention. According to one embodiment of the invention, the TTR promoter is used in the expression cassette of the present invention.

In some embodiments of the invention, the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid that encodes the FIX protein (coagulation factor IX);
an hGH1 polyadenylation signal (human growth hormone gene polyadenylation signal);
a right-hand (second) ITR.

In some embodiments of the invention, the left-hand (first) ITR has the following nucleic acid sequence:

Cctgcaggcagctgcgcgctcgctcgctcactgaggccgcccgggcgtcgggcgacctttggtcgcccggcctcagtgagcgagcgag
cgcgcagagagggagtggccaactccatcactaggggttcct (SEQ ID NO: 6).

In some embodiments of the invention, the TTR promoter has the following nucleic acid sequence:

tcgagcttgggctgcaggtcgagggcactgggaggatgttgagtaagatggaaaactactgatgacccttgcagagacagagtattagga
catgtttgaacaggggccggcgatcagcaggtagctctagaggatccccgtctgtctgcacatttcgtagagcgagtgttccgatactctaat
ctccctaggcaaggttcatatttgtgtaggttacttattctccttttgttgactaagtcaataatcagaatcagcaggtttggagtcagcttggcag
ggatcagcagcctgggttggaaggagggggtataaaagccccttcaccaggagaagccgtcacacagatccacaagctcctgacagga
agct (SEQ ID NO: 7).

In some embodiments of the invention, the intron of the hBG1 gene has the following nucleic acid sequence:

cgaatcccggccgggaacggtgcattggaacgcggattccccgtgccaagagtgacgtaagtaccgcctatagagtctataggcccacaa
aaaatgctttcttcttttaatatactttttttgtttatcttatttctaatactttccctaatctctttctttcagggcaataatgatacaatgtatcatgcctcttt
gcaccattctaaagaataacagtgataatttctgggttaaggcaatagcaatatttctgcatataaatatttctgcatataaattgtaactgatgtaa
gaggtttcatattgctaatagcagctacaatccagctaccattctgcttttattttatggttgggataaggctggattattctgagtccaagctagg
ccctttttgctaatcatgttcatacctcttatcttcctcccacagctcctgggcaacgtgctggtctgtgtgctggcccatcactttggcaaagaatt
gggat (SEQ ID NO: 8).

In some embodiments of the invention, the hGH1 polyadenylation signal has the following nucleic acid sequence:

acgggtggcatccctgtgacccctccccagtgccCctcctggccctggaagttgccactccagtgcccaccagccttgtcctaataaaatta
agttgcatcattttgtctgactaggtgtccttctataatattatggggtggaggggggtggtatggagcaaggggcaagttgggaagacaacc
tgtagggcctgcggggtctattgggaaccaagctggagtgcagtggcacaatcttggctcactgcaatctccgcctcctgggttcaagcgat
tctcctgcctcagcctcccgagttgttgggattccaggcatgcatgaccaggctcagctaatttttgtttttttggtagagacggggtttcaccat
attggccaggctggtctccaactcctaatctcaggtgatctacccaccttggcctcccaaattgctgggattacaggcgtgaaccactgctcc
cttccctgtcctt (SEQ ID NO: 9).

In some embodiments of the invention, the right-hand (second) ITR has the following nucleic acid sequence:

aggaacccctagtgatggagttggccactccctctctgcgcgctcgctcgctcactgaggccgggcgaccaaaggtcgcccgacgcccg
ggctttgcccgggcggcctcagtgagcgagcgagcgcgcagctgcctgcagg (SEQ ID NO: 10).

In some embodiments of the invention, the expression cassette includes a nucleotide sequence that is selected from the group comprising: 
cctgcaggcagctgcgcgctcgctcgctcactgaggccgcccgggcgtcgggcgacctttggtcgcccggcctcagtgagcg
agcgagcgcgcagagagggagtggccaactccatcactaggggttcctgcggccgcacgcgtgccgccaccATGGtcgagcttgg
 
gctgcaggtcgagggcactgggaggatgttgagtaagatggaaaactactgatgacccttgcagagacagagtattaggacatgtttgaac
aggggccggcgatcagcaggtagctctagaggatccccgtctgtctgcacatttcgtagagcgagtgttccgatactctaatctccctaggc
aaggttcatatttgtgtaggttacttattctccttttgttgactaagtcaataatcagaatcagcaggtttggagtcagcttggcagggatcagcag
cctgggttggaaggagggggtataaaagccccttcaccaggagaagccgtcacacagatccacaagctcctgacaggaagctctaggtg
actctcttaaggtagcctccgcggattcgaatcccggccgggaacggtgcattggaacgcggattccccgtgccaagagtgacgtaagtac
cgcctatagagtctataggcccacaaaaaatgctttcttcttttaatatactttttttgtttatcttatttctaatactttccctaatctctttctttcagggc
aataatgatacaatgtatcatgcctctttgcaccattctaaagaataacagtgataatttctgggttaaggcaatagcaatatttctgcatataaata
tttctgcatataaattgtaactgatgtaagaggtttcatattgctaatagcagctacaatccagctaccattctgcttttattttatggttgggataag
gctggattattctgagtccaagctaggcccttttgctaatcatgttcatacctcttatcttcctcccacagctcctgggcaacgtgctggtctgtgt
gctggcccatcactttggcaaagaattgggattcgaacatCGATATGCAGCGGGTCAACATGATCATGGCGG
AGTCGCCGGGCCTGATCACGATCTGCCTCCTCGGGTACCTGCTCTCCGCCGAGTGCA
CCGTGTTCCTGGACCACGAGAACGCCAACAAGATCCTCAACCGGCCCAAGCGCTAC
AACTCCGGCAAGCTGGAGGAGTTCGTGCAGGGGAACCTCGAGCGCGAGTGCATGGA
GGAGAAGTGCTCGTTCGAGGAGGCGCGGGAGGTGTTCGAGAACACCGAGCGCACCA
CGGAGTTCTGGAAGCAGTACGTGGACGGGGACCAGTGCGAGTCGAACCCGTGCCTC
AACGGGGGGTCGTGCAAGGACGACATCAACTCGTACGAGTGCTGGTGCCCCTTCGG
CTTCGAGGGCAAGAACTGCGAGCTGGACGTGACCTGCAACATCAAGAACGGGCGCT
GCGAGCAGTTCTGCAAGAACAGCGCCGACAACAAGGTGGTCTGCTCCTGCACCGAG
GGGTACCGCCTCGCGGAGAACCAGAAGTCCTGCGAGCCGGCCGTGCCCTTCCCCTG
CGGCCGCGTGTCCGTCAGCCAGACGTCGAAGCTGACGCGCGCCGAGACCGTCTTCC
CGGACGTGGACTACGTGAACTCGACGGAGGCCGAGACCATCCTGGACAACATCACC
CAGAGCACCCAGTCCTTCAACGACTTCACGCGGGTGGTCGGCGGCGAGGACGCCAA
GCCCGGGCAGTTCCCGTGGCAGGTCGTCCTCAACGGGAAGGTCGACGCGTTCTGCG
GCGGGAGCATCGTGAACGAGAAGTGGATCGTGACCGCCGCGCACTGCGTCGAGACG
GGCGTGAAGATCACCGTGGTGGCCGGGGAGCACAACATCGAGGAGACGGAGCACA
CCGAGCAGAAGCGGAACGTGATCCGCATCATCCCGCACCACAACTACAACGCCGCC
ATCAACAAGTACAACCACGACATCGCGCTCCTCGAGCTGGACGAGCCGCTGGTCCT
CAACTCCTACGTCACGCCGATCTGCATCGCCGACAAGGAGTACACGAACATCTTCCT
GAAGTTCGGGAGCGGCTACGTCTCGGGCTGGGGCCGCGTGTTCCACAAGGGGCGCA
GCGCGCTCGTGCTCCAGTACCTGCGGGTCCCCCTGGTCGACCGCGCGACCTGCCTCC
TCTCCACGAAGTTCACGATCTACAACAACATGTTCTGCGCGGGGTTCCACGAGGGCG
GCCGGGACAGCTGCCAGGGCGACAGCGGGGGCCCGCACGTGACGGAGGTGGAGGG
CACGAGCTTCCTGACCGGGATCATCTCGTGGGGCGAGGAGTGCGCGATGAAGGGGA
AGTACGGCATCTACACCAAGGTCAGCCGGTACGTGAACTGGATCAAGGAGAAGACG
AAGCTGACGTGATGAAgatctacgggtggcatccctgtgacccctccccagtgccCctcctggccctggaagttgccactc
 
cagtgcccaccagccttgtcctaataaaattaagttgcatcattttgtctgactaggtgtccttctataatattatggggtggaggggggtggtat
ggagcaaggggcaagttgggaagacaacctgtagggcctgcggggtctattgggaaccaagctggagtgcagtggcacaatcttggctc
actgcaatctccgcctcctgggttcaagcgattctcctgcctcagcctcccgagttgttgggattccaggcatgcatgaccaggctcagctaat
ttttgtttttttggtagagacggggtttcaccatattggccaggctggtctccaactcctaatctcaggtgatctacccaccttggcctcccaaatt
gctgggattacaggcgtgaaccactgctcccttccctgtccttctgattttgtaggtaaccacgtgcggaccgagcggccgcaggaacccct
agtgatggagttggccactccctctctgcgcgctcgctcgctcactgaggccgggcgaccaaaggtcgcccgacgcccgggctttgcccg
ggcggcctcagtgagcgagcgagcgcgcagctgcctgcagg (SEQ ID NO: 3)
 or

cctgcaggcagctgcgcgctcgctcgctcactgaggccgcccgggcgtcgggcgacctttggtcgcccggcctcagtgagcg
agcgagcgcgcagagagggagtggccaactccatcactaggggttcctgcggccgcacgcgtgccgccaccATGGtcgagcttgg
gctgcaggtcgagggcactgggaggatgttgagtaagatggaaaactactgatgacccttgcagagacagagtattaggacatgtttgaac
aggggccggcgatcagcaggtagctctagaggatccccgtctgtctgcacatttcgtagagcgagtgttccgatactctaatctccctaggc
aaggttcatatttgtgtaggttacttattctccttttgttgactaagtcaataatcagaatcagcaggtttggagtcagcttggcagggatcagcag
cctgggttggaaggagggggtataaaagccccttcaccaggagaagccgtcacacagatccacaagctcctgacaggaagctctaggtg
actctcttaaggtagcctccgcggattcgaatcccggccgggaacggtgcattggaacgcggattccccgtgccaagagtgacgtaagtac
cgcctatagagtctataggcccacaaaaaatgctttcttcttttaatatacttttttgtttatcttatttctaatactttccctaatctctttctttcagggc
aataatgatacaatgtatcatgcctctttgcaccattctaaagaataacagtgataatttctgggttaaggcaatagcaatatttctgcatataaata
tttctgcatataaattgtaactgatgtaagaggtttcatattgctaatagcagctacaatccagctaccattctgcttttattttatggttgggataag
gctggattattctgagtccaagctaggcccttttgctaatcatgttcatacctcttatcttcctcccacagctcctgggcaacgtgctggtctgtgt
gctggcccatcactttggcaaagaattgggattcgaacatcgatataaATGCAGCGGGTGAACATGATCATGGCC
GAGTCCCCAGGCCTGATTACCATCTGTCTGCTGGGCTACCTGCTGAGCGCCGAATGC
ACCGTGTTTCTGGACCACGAGAACGCCAACAAGATCCTGAACCGCCCTAAGCGGTA
CAACTCCGGCAAGCTGGAGGAGTTTGTGCAGGGCAATCTGGAGCGGGAGTGTATGG
AGGAGAAGTGCAGCTTCGAGGAGGCCAGGGAGGTGTTCGAGAACACCGAGAGGAC
CACCGAGTTCTGGAAGCAGTATGTGGACGGCGACCAGTGCGAGTCTAATCCTTGTCT
GAATGGCGGGAGCTGCAAGGACGACATCAACAGCTACGAGTGCTGGTGCCCTTTCG
GCTTCGAGGGCAAGAATTGCGAGCTGGACGTGACCTGCAACATCAAGAACGGCCGG
TGTGAGCAGTTCTGCAAGAACAGCGCCGACAACAAGGTGGTGTGCTCCTGCACCGA
GGGATACAGGCTGGCCGAGAATCAGAAGAGCTGTGAGCCCGCCGTGCCATTCCCCT
GTGGCAGAGTGTCTGTGAGCCAGACCAGCAAGCTGACCAGAGCCGAGACCGTGTTT
CCAGACGTGGACTACGTGAACAGCACCGAGGCCGAGACCATCCTGGATAATATCAC
CCAGTCCACCCAGAGCTTCAACGACTTCACCAGAGTGGTGGGAGGCGAGGATGCCA
AGCCAGGACAGTTCCCTGGCAGGTGGTGCTGAATGGCAAGGTGGACGCCTTCTGC
GGAGGCAGCATCGTGAACGAGAAGTGGATTGTGACCGCAGCCCACTGCGTGGAGAC
TGGCGTGAAGATTACCGTGGTCGCCGGCGAGCACAATATCGAAGAGACCGAGCACA

CCGAGCAGAAGCGCAACGTGATCCGGATCATCCCTCACCACAACTACAACGCAGCC
ATCAACAAGTACAACCACGACATCGCCCTGCTGGAGCTGGACGAGCCACTGGTGCT
GAACTCTTACGTGACCCCTATCTGCATCGCCGACAAGGAGTACACCAACATCTTCCT
GAAGTTCGGCAGCGGCTACGTGAGCGGATGGGGCAGAGTGTTCACAAGGGCAGGA
GCGCCCTGGTGCTGCAGTATCTGAGAGTGCCACTGGTGGACAGAGCTACCTGCCTGC
TGAGCACCAAGTTCACCATCTACAACAACATGTTCTGCGCCGGCTTCCACGAGGGGG
GAAGAGACTCTTGCCAGGGCGATTCCGGCGGACCACACGTGACCGAAGTGGAGGGC
ACCAGCTTCCTGACCGGCATCATCTCCTGGGGCGAGGAATGCGCCATGAAGGGCAA
GTACGGCATCTACACCAAGGTGAGCAGGTACGTGAACTGGATCAAGGAGAAGACCA
AGCTGACCtgaagatctacgggtggcatccctgtgacccctccccagtgccCctcctggccctggaagttgccactccagtgccca
ccagccttgtcctaataaaattaagttgcatcattttgtctgactaggtgtccttctataatattatggggtggaggggggtggtatggagcaag
gggcaagttgggaagacaacctgtagggcctgcggggtctattgggaaccaagctggagtgcagtggcacaatcttggctcactgcaatct
ccgcctcctgggttcaagcgattctcctgcctcagcctcccgagttgttgggattccaggcatgcatgaccaggctcagctaatttttgttttttt
ggtagagacggggtttcaccatattggccaggctggtctccaactcctaatctcaggtgatctacccaccttggcctcccaaattgctgggatt
acaggcgtgaaccactgctcccttccctgtccttctgattttgtaggtaaccacgtgcggaccgagcggccgcaggaacccctagtgatgga
gttggccactccctctctgcgcgctcgctcgctcactgaggccgggcgaccaaaggtcgcccgacgcccgggctttgcccgggcggcct
cagtgagcgagcgagcgcgcagctgcctgcagg (SEQ ID NO: 5).

In one aspect, the present invention relates to an expression vector that includes the above codon-optimized nucleic acid or any of the above expression cassettes.

In some embodiments of the invention, the vector is a plasmid, i.e., a circular double stranded piece of DNA into which additional DNA segments may be ligated.

In some embodiments of the invention, the vector is a viral vector, wherein additional DNA segments may be ligated into the viral genome.

In some embodiments of the invention, vectors are capable of autonomous replication in a host cell into which they are introduced (e.g. bacterial vectors having a bacterial origin site of replication and episomal mammalian vectors). In further embodiments of the invention, vectors (e.g. non-episomal mammalian vectors) may be integrated into the genome of a host cell upon introduction into a host cell, and thereby are replicated along with the host gene. Moreover, certain vectors are capable of directing the expression of genes to which they are operably linked. Such vectors are referred to herein as "recombinant expression vectors" (or simply, "expression vectors").

Expression vectors include plasmids, retroviruses, adenoviruses, adeno-associated viruses (AAVs), plant viruses, such as cauliflower mosaic virus, tobacco mosaic virus, cosmids, YACs, EBV derived episomes, and the like. DNA molecules may be ligated into a vector such that transcriptional and translational control sequences within the vector serve their intended function of regulating the transcription and translation of the DNA. An expression vector and expression control sequences may be chosen to be compatible with the expression host cell used. DNA molecules may be introduced into the expression vector by standard methods (e.g. ligation of complementary restriction sites, or blunt end ligation if no restriction sites are present).

The recombinant expression vector may also encode a leader peptide (or a signal peptide) that facilitates the secretion of the protein of interest from a host cell. The gene of the protein of interest may be cloned into the vector such that the signal peptide is linked in-frame to the amino terminus of the protein of interest. The leader peptide (or signal peptide) may be an immunoglobulin leader peptide or other leader peptide (that is, a non-immunoglobulin protein leader peptide).

In addition to the FIX gene of the present invention, the recombinant expression of the vectors of the present invention may carry regulatory sequences that control the expression of the FIX gene in a host cell. It will be understood by those skilled in the art that the design of an expression vector, including the selection of regulatory sequences, may depend on such factors as the choice of a host cell to be transformed, the level of expression of a desired protein, and so forth. Preferred control sequences for an expression host cell in mammals include viral elements that ensure high levels of protein expression in mammalian cells, such as promoters and/or enhancers derived from a retroviral LTR, cytomegalovirus (CMV) (such as a CMV promoter/enhancer), simian virus 40 (SV40) (such as a SV40 promoter/enhancer), adenovirus, (e.g. the major late promoter adenovirus (AdMLP)), polyomavirus and strong mammalian promoters such as TTR promoter, native immunoglobulin promoter or actin promoter.

The term "control sequences" refers to DNA sequences necessary for the expression of an operably linked coding sequence in a particular host organism. The control sequences that are suitable for prokaryotes are, for example, a promoter, optionally an operator sequence and a ribosome binding site. Eukaryotic cells are known to include promoters, polyadenylation signals, and enhancers.

As used in the present description, the term "promoter" or "transcription regulatory sequence" or "regulatory sequence" refers to a nucleic acid fragment that controls the transcription of one or more coding sequences, and that is located upstream with respect to the direction of reading relative to the direction of transcription from the transcription initiation site of the coding sequence, and is structurally identified by the presence of a binding site for DNA-dependent RNA polymerase, transcription initiation sites and any other DNA sequences, including, but not limited to, transcription factor binding sites, repressor and activator protein binding sites, and any other sequences of nucleotides known to one of skill in the art that directly or indirectly regulate the level of transcription with said promoter. A "constitutive" promoter is a promoter that is active in most tissues under typical physiological and developmental conditions. An "inducible" promoter is a promoter that is physiologically or developmentally regulated, e.g. under the influence of a chemical inducer. A "tissue specific" promoter is only active in specific types of tissues or cells.

The terms "enhancers" or "enhancer" as used herein may refer to a DNA sequence that is located adjacent to the DNA sequence that encodes a recombinant product. Enhancer elements are typically located in a 5' direction from a promoter element or can be located downstream of or within a coding DNA sequence (e.g. a DNA sequence transcribed or translated into a recombinant product or products). Hence, an enhancer element may be located 100 base pairs, 200 base pairs, or 300 or more base pairs upstream of a DNA sequence that encodes a recombinant product, or downstream of said sequence. Enhancer elements may increase the amount of a recombinant product being expressed from a DNA sequence above the level of expression associated with a single promoter element. Multiple enhancer elements are readily available to those of ordinary skill in the art.

In addition to the above genes and regulatory sequences, recombinant expression vectors of the invention may carry additional sequences, such as sequences that regulate replication of a vector in host cells (e.g. origins of replication) and selectable marker genes. The selectable marker gene facilitates the selection of host cells into which a vector has been introduced (see e.g., U.S. Patent Nos. 4,399,216, 4,634,665 and 5,179,017). For example, the selectable marker gene typically confers resistance to medicinal agents, such as G418, ampicillin, hygromycin or methotrexate, on a host cell into which the vector has been introduced. For example, selectable marker genes include a dihydrofolate reductase (DHFR) gene (for use in dhfr-host cells during methotrexate selection/amplification), a neo gene (for G418 selection), and a glutamate synthetase gene.

The term "expression control sequence" as used in the present description refers to polynucleotide sequences that are necessary to effect the expression and processing of coding sequences to which they are ligated. Expression control sequences include appropriate transcription initiation, termination, promoter and enhancer sequences; efficient RNA processing signals such as splicing and polyadenylation signals; sequences that stabilize cytoplasmic mRNA; sequences that enhance translation efficiency (i.e., Kozak consensus sequence); sequences that enhance protein stability; and when desired, sequences that enhance protein secretion. The nature of such control sequences differs depending upon the host organism; in prokaryotes, such control sequences generally include the promoter of ribosome binding site, and transcription termination sequences; in eukaryotes, typically, such control sequences include promoters and transcription termination sequences. The term "control sequences" includes at least all components, the presence of which is essential for expression and processing, and can also include additional components, the presence of which is advantageous, for example, leader sequences and fusion partner sequences.

As used herein, the term "operably linked" refers to a linkage of polynucleotide (or polypeptide) elements in a functional relationship. A nucleic acid is "operably linked" when it is present in functional relationship conditions with another nucleic acid sequence. For example, a transcription regulatory sequence is operably linked to a coding sequence if it affects the transcription of said coding sequence. The term "operably linked" means that the DNA sequences being linked are typically contiguous and, where it is necessary to join two protein coding regions, are also contiguous and are present in the reading frame.

In one embodiment of the present invention, "expression vector" relates to a vector comprising one or more polynucleotide sequences of interest, genes of interest, or "transgenes" that are flanked by parvoviral sequences or inverted terminal repeat (ITR) sequences.

Neither the cassette nor the vector of the invention comprises nucleotide sequences of genes encoding non-structural proteins (Rep) and structural proteins (Cap) of the adeno-associated virus.

### AAV5 (adeno-associated virus serotype 5)-based recombinant virus

In one aspect, the present invention relates to an isolated AAV5 (adeno-associated virus serotype 5)-based recombinant virus for increasing the FIX gene expression in target cells, comprising the above codon-optimized nucleic acid or any of the above expression cassettes.

The term "AAV-based recombinant virus" (or "AAV-based virus-like particle", or "AAV recombinant virus strain", or "AAV recombinant vector", or "rAAV vector") as used in this description refers to the above expression cassette (or the above expression vector), which is enclosed within the AAV capsid.

The Cap gene, among other alternative products, encodes 3 capsid proteins (VP1, VP2, and VP3). VP1, VP2, and VP3 are present at 1:1:10 ratio to form an icosahedral capsid (Xie Q. et al. The atomic structure of adeno-associated virus (AAV-2), a vector for human gene therapy. Proc Natl Acad Sci USA, 2002; 99:10405-10410). Transcription of these genes starts from a single promoter, p40. The molecular weights of the corresponding proteins (VP1, VP2 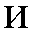 VP3) are 87, 72, and 62 kDa, respectively. All of the three proteins are translated from a single mRNA. Following transcription, pre-mRNA may be spliced in two different manners, where either longer or shorter intron is excised to form mRNAs of various nucleotide lengths.

During the production of the AAV (rAAV)-based recombinant virus, an expression cassette flanked by ITR is packaged into an AAV capsid. The genes required for AAV replication, as mentioned above, are not included in the cassette.

The expression cassette DNA is packaged into a viral capsid in the form of a single stranded DNA molecule (ssDNA) being approximately 3000 nucleotides long. Once a cell is infected with the virus, the single-stranded DNA is converted to the form of double-stranded DNA (dsDNA). The dsDNA can only be used by the cell's proteins, which transcribe the present gene or genes into RNA.

In some embodiments of the invention, the AAV5-based recombinant virus has a capsid that includes the AAV5 protein VP1.

In some embodiments of the invention, the AAV5-based recombinant virus has a capsid that includes the AAV5 protein VP1 having the amino acid sequence

MSFVDHPPDWLEEVGEGLREFLGLEAGPPKPKPNQQHQDQARGLVLPGYNYLGPGNGL
DRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG
NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPETGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRTTRPIGTRYLTRPL (SEQ ID NO: 11).

In some embodiments of the invention, the AAV5-based recombinant virus has a capsid that includes the AAV5 protein VP2.

In some embodiments of the invention, the AAV5-based recombinant virus has a capsid that includes the AAV5 protein VP2 having the following amino acid sequence:

TAPTGKRIDDHFPKRKKARTEEDSKPSTSSDAEAGPSGSQQLQIPAQPASSLGADTMSAG
GGGPLGDNNQGADGVGNASGDWHCDSTWMGDRVVTKSTRTWVLPSYNNHQYREIKS
GSVDGSNANAYFGYSTPWGYFDFNRFHSHWSPRDWQRLINNYWGFRPRSLRVKIFNIQ
VKEVTVQDSTTTIANNLTSTVQVFTDDDYQLPYVVGNGTEGCLPAFPPQVFTLPQYGYA
TLNRDNTENPTERSSFFCLEYFPSKMLRTGNNFEFTYNFEEVPFHSSFAPSQNLFKLANPL
VDQYLYRFVSTNNTGGVQFNKNLAGRYANTYKNWFPGPMGRTQGWNLGSGVNRASV
SAFATTNRMELEGASYQVPPQPNGMTNNLQGSNTYALENTMIFNSQPANPGTTATYLE
GNMLITSESETQPVNRVAYNVGGQMATNNQSSTTAPATGTYNLQEIVPGSVWMERDVY
LQGPIWAKIPETGAHFHPSPAMGGFGLKHPPPMMLIKNTPVPGNITSFSDVPVSSFITQYS

TGQVTVEMEWELKKENSKRWNPEIQYTNNYNDPQFVDFAPDSTGEYRTTRPIGTRYLT
RPL (SEQ ID NO: 12).

In some embodiments of the invention, the AAV5-based recombinant virus has a capsid that includes the AAV5 protein VP3.

In some embodiments of the invention, the AAV5-based recombinant virus has a capsid that includes the AAV5 protein VP3 having the following amino acid sequence

MSAGGGGPLGDNNQGADGVGNASGDWHCDSTWMGDRVVTKSTRTWVLPSYNNHQY
REIKSGSVDGSNANAYFGYSTPWGYFDFNRFHSHWSPRDWQRLINNYWGFRPRSLRVK
IFNIQVKEVTVQDSTTTIANNLTSTVQVFTDDDYQLPYVVGNGTEGCLPAFPPQVFTLPQ
YGYATLNRDNTENPTERSSFFCLEYFPSKMLRTGNNFEFTYNFEEVPFHSSFAPSQNLFK
LANPLVDQYLYRFVSTNNTGGVQFNKNLAGRYANTYKNWFPGPMGRTQGWNLGSGV
NRASVSAFATTNRMELEGASYQVPPQPNGMTNNLQGSNTYALENTMIFNSQPANPGTT
ATYLEGNMLITSESETQPVNRVAYNVGGQMATNNQSSTTAPATGTYNLQEIVPGSVWM
ERDVYLQGPIWAKIPETGAHFHPSPAMGGFGLKHPPPMMLIKNTPVPGNITSFSDVPVSS
FITQYSTGQVTVEMEWELKKENSKRWNPEIQYTNNYNDPQFVDFAPDSTGEYRTTRPIG
TRYLTRPL (SEQ ID NO: 13).

In some embodiments of the invention, the AAV5-based recombinant virus has a capsid that includes the AAV5 proteins VP1, VP2, and VP3.

In some embodiments of the invention, the AAV5-based recombinant virus has a capsid that includes the proteins VP1 having the amino acid sequence of SEQ ID NO: 11, VP2 having the amino acid sequence of SEQ ID NO: 12, and VP3 having the amino acid sequence of SEQ ID NO: 13.

In some embodiments of the invention, the AAV5-based recombinant virus has a capsid that includes the AAV5 protein VP1 having the amino acid sequence of SEQ ID NO: 11 with one or more point mutations.

In some embodiments of the invention, the AAV5-based recombinant virus has a capsid that includes an AAV5 protein VP1 having an amino acid sequence that includes amino acid substitutions at positions S2A and T711S of wild-type AAV5 VP1 (SEQ ID NO: 11), and has the amino acid sequence

MAFVDHPPDWLEEVGEGLREFLGLEAGPPKPKPNQQHQDQARGLVLPGYNYLGPGNG
LDRGEPVNRADEVAREHDISYNEQLEAGDNPYLKYNHADAEFQEKLADDTSFGGNLGK
AVFQAKKRVLEPFGLVEEGAKTAPTGKRIDDHFPKRKKARTEEDSKPSTSSDAEAGPSG
SQQLQIPAQPASSLGADTMSAGGGGPLGDNNQGADGVGNASGDWHCDSTWMGDRVV
TKSTRTWVLPSYNNHQYREIKSGSVDGSNANAYFGYSTPWGYFDFNRFHSHWSPRDW
QRLINNYWGFRPRSLRVKIFNIQVKEVTVQDSTTTIANNLTSTVQVFTDDDYQLPYVVG

NGTEGCLPAFPPQVFTLPQYGYATLNRDNTENPTERSSFFCLEYFPSKMLRTGNNFEFTY
NFEEVPFHSSFAPSQNLFKLANPLVDQYLYRFVSTNNTGGVQFNKNLAGRYANTYKNW
FPGPMGRTQGWNLGSGVNRASVSAFATTNRMELEGASYQVPPQPNGMTNNLQGSNTY
ALENTMIFNSQPANPGTTATYLEGNMLITSESETQPVNRVAYNVGGQMATNNQSSTTAP
ATGTYNLQEIVPGSVWMERDVYLQGPIWAKIPETGAHFHPSPAMGGFGLKHPPPMMLI
KNTPVPGNITSFSDVPVSSFITQYSTGQVTVEMEWELKKENSKRWNPEIQYTNNYNDPQ
FVDFAPDSTGEYRSTRPIGTRYLTRPL (SEQ ID NO: 14).

In some embodiments of the invention, the AAV5-based recombinant virus has a capsid that includes the AAV5 protein VP2 having the amino acid sequence of SEQ ID NO: 12 with one or more point mutations.

In some embodiments of the invention, the AAV5-based recombinant virus has a capsid that includes an AAV5 protein VP2 having an amino acid sequence that includes an amino acid substitution at position T575S of wild-type AAV5 VP2 (SEQ ID NO: 12), and has the amino acid sequence

TAPTGKRIDDHFPKRKKARTEEDSKPSTSSDAEAGPSGSQQLQIPAQPASSLGADTMS
AGGGGPLGDNNQGADGVGNASGDWHCDSTWMGDRVVTKSTRTWVLPSYNNHQY
REIKSGSVDGSNANAYFGYSTPWGYFDFNRFHSHWSPRDWQRLINNYWGFRPRSLR
VKIFNIQVKEVTVQDSTTTIANNLTSTVQVFTDDDYQLPYVVGNGTEGCLPAFPPQV
FTLPQYGYATLNRDNTENPTERSSFFCLEYFPSKMLRTGNNFEFTYNFEEVPFHSSFA
PSQNLFKLANPLVDQYLYRFVSTNNTGGVQFNKNLAGRYANTYKNWFPGPMGRTQ
GWNLGSGVNRASVSAFATTNRMELEGASYQVPPQPNGMTNNLQGSNTYALENTMI
FNSQPANPGTTATYLEGNMLITSESETQPVNRVAYNVGGQMATNNQSSTTAPATGT
YNLQEIVPGSVWMERDVYLQGPIWAKIPETGAHFHPSPAMGGFGLKHPPPMMLIKN
TPVPGNITSFSDVPVSSFITQYSTGQVTVEMEWELKKENSKRWNPEIQYTNNYNDPQ
FVDFAPDSTGEYRSTRPIGTRYLTRPL (SEQ ID NO: 15).

In some embodiments of the invention, the AAV5-based recombinant virus has a capsid that includes the AAV5 protein VP3 having the amino acid sequence of SEQ ID NO: 13 with one or more point mutations.

In some embodiments of the invention, the AAV5-based recombinant virus has a capsid that includes an AAV5 protein VP3 having an amino acid sequence that includes an amino acid substitution at position T519S of wild-type AAV5 VP3 (SEQ ID NO: 13), and has the amino acid sequence

MSAGGGGPLGDNNQGADGVGNASGDWHCDSTWMGDRVVTKSTRTWVLPSYNNHQY
REIKSGSVDGSNANAYFGYSTPWGYFDFNRFHSHWSPRDWQRLINNYWGFRPRSLRVK
IFNIQVKEVTVQDSTTTIANNLTSTVQVFTDDDYQLPYVVGNGTEGCLPAFPPQVFTLPQ

YGYATLNRDNTENPTERSSFFCLEYFPSKMLRTGNNFEFTYNFEEVPFHSSFAPSQNLFK
LANPLVDQYLYRFVSTNNTGGVQFNKNLAGRYANTYKNWFPGPMGRTQGWNLGSGV
NRASVSAFATTNRMELEGASYQVPPQPNGMTNNLQGSNTYALENTMIFNSQPANPGTT
ATYLEGNMLITSESETQPVNRVAYNVGGQMATNNQSSTTAPATGTYNLQEIVPGSVWM
ERDVYLQGPIWAKIPETGAHFHPSPAMGGFGLKHPPPMMLIKNTPVPGNITSFSDVPVSS
FITQYSTGQVTVEMEWELKKENSKRWNPEIQYTNNYNDPQFVDFAPDSTGEYRSTRPIG
TRYLTRPL (SEQ ID NO: 16).

In some embodiments of the invention, the AAV5-based recombinant virus has a capsid that includes the proteins VP1 having the amino acid sequence of SEQ ID NO: 11 with one or more point mutations, VP2 having the amino acid sequence of SEQ ID NO: 12 with one or more point mutations, and VP3 having the amino acid sequence of SEQ ID NO: 13 with one or more point mutations.

In some embodiments of the invention, the AAV5-based recombinant virus has a capsid that includes the proteins VP1 having the amino acid sequence of SEQ ID NO: 14, VP2 having the amino acid sequence of SEQ ID NO: 15 and VP3 having the amino acid sequence of SEQ ID NO: 16.

The phrase "more point mutations" refers to two, three, four, five, six, seven, eight, nine, or ten point substitutions.

Particularly preferred embodiments include substitutions (mutations) that are conservative in nature, i.e. substitutions that take place within a family of amino acids that are joined in their side chains. In particular, amino acids are typically divided into four families: (1) acidic amino acids are aspartate and glutamate; (2) basic amino acids are lysine, arginine, histidine; (3) nonpolar amino acids are alanine, valine, leucine, isoleucine, proline, phenylalanine, methionine, tryptophan, and (4) uncharged polar amino acids are glycine, asparagine, glutamine, cysteine, serine, threonine, tyrosine. Phenylalanine, tryptophan, and tyrosine are sometimes classified as aromatic amino acids. For example, it is reasonably predictable that an isolated substitution of leucine for isoleucine or valine, an aspartate for a glutamate, a threonine for a serine, or a similar conservative substitution of an amino acid for a structurally related amino acid, will not have a major effect on the biological activity. For example, the polypeptide of interest may include up to about 5-10 conservative or non-conservative amino acid substitutions, so long as the desired function of the molecule remains intact.

A variant of point mutations in the sequences of AAV5 proteins VP1, VP2, or VP3 using amino acid substitutions is a substitution of at least one amino acid residue in the AAV5 protein VP1, VP2, or VP3 with another amino acid residue.

Conservative substitutions are shown in Table A under "preferred substitutions".

| Table A | | |
|---|---|---|
| Initial residue | Exemplary substitutions | Preferred substitutions |
| Ala (A) | Val; Leu; Ile | Val |
| Arg(R) | Lys; Gin; Asn | Lys |
| Asn(N) | Gin; His; Asp, Lys; Arg | Gin |
| Asp (D) | Glu; Asn | Glu |
| Cys (C) | Ser; Ala | Ser |
| Gln(Q) | Asn; Glu | Asn |
| Glu (E) | Asp; Gin | Asp |
| Gly(G) | Ala | Ala |
| His (H) | Asn; Gin; Lys; Arg | Arg |
| Ile (I) | Leu; Val; Met; Ala; Phe; Norleucine | Leu |
| Leu (L) | Norleucine; Ile; Val; Met; Ala; Phe | Ile |
| Lys (K) | Arg; Gin; Asn | Arg |
| Met (M) | Leu; Phe; Ile | Leu |
| Phe(F) | Trp; Leu; Val; Ile; Ala; Tyr | Tyr |
| Pro (P) | Ala | Ala |
| Ser(S) | Thr | Thr |
| Thr (T) | Val; Ser | Ser |
| Trp(W) | Tyr; Phe | Tyr |
| Tyr(Y) | Trp; Phe; Thr; Ser | Phe |
| Val (V) | Ile; Leu; Met; Phe; Ala; Norleucine | Leu |

In some embodiments of the invention, the AAV5-based recombinant virus has a capsid that includes the AAV5 protein VP1 having the amino acid sequence of SEQ ID NO: 11 or the amino acid sequence of SEQ ID NO: 11 with one or more point mutations, and the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments of the invention, the AAV5-based recombinant virus has a capsid that includes the AAV5 protein VP1 having the amino acid sequence of SEQ ID NO: 11, the AAV5 protein VP2 having the amino acid sequence of SEQ ID NO: 12, and the AAV5 protein VP3 having the amino acid sequence of SEQ ID NO: 13, and the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments of the invention, the AAV5-based recombinant virus has a capsid that includes the AAV5 protein VP1 having the amino acid sequence of SEQ ID NO: 11 with one or more point mutations, the AAV5 protein VP2 having the amino acid sequence of SEQ ID NO: 12 with one or more point mutations, and the AAV5 protein VP3 having the amino acid sequence of SEQ ID NO: 13 with one or more point mutations, and the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments of the invention, the AAV5-based recombinant virus has a capsid that includes the AAV5 protein VP1 having the amino acid sequence of SEQ ID NO: 14, the AAV5 protein VP2 having the amino acid sequence of SEQ ID NO: 15, and the AAV5 protein VP3 having the amino acid sequence of SEQ ID NO: 16, and the expression cassette includes the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the above codon-optimized nucleic acid;
an hGH1 polyadenylation signal (human growth hormone gene polyadenylation signal);
a right-hand (second) ITR.

In some embodiments of the invention, the AAV5-based recombinant virus has a capsid that includes the AAV5 protein VP1 having the amino acid sequence of SEQ ID NO: 11 or the amino acid sequence of SEQ ID NO: 11 with one or more point mutations, and the expression cassette includes a nucleotide sequence selected from the group comprising: SEQ ID NO: 3 or SEQ ID NO: 5.

In some embodiments of the invention, the AAV5-based recombinant virus has a capsid that includes the proteins VP1 having the amino acid sequence of SEQ ID NO: 11, VP2 having the amino acid sequence of SEQ ID NO: 12, and VP3 having the amino acid sequence of SEQ ID NO: 13, and the expression cassette includes a nucleotide sequence selected from the group comprising: SEQ ID NO: 3 or SEQ ID NO: 5.

In some embodiments of the invention, the AAV5-based recombinant virus has a capsid that includes the proteins VP1 having the amino acid sequence of SEQ ID NO: 11 with one or more point mutations, VP2 having the amino acid sequence of SEQ ID NO: 12 with one or more point mutations, and VP3 having the amino acid sequence of SEQ ID NO: 13 with one or more point mutations, and the expression cassette includes a nucleotide sequence selected from the group comprising: SEQ ID NO: 3 or SEQ ID NO: 5.

In some embodiments of the invention, the AAV5-based recombinant virus has a capsid that includes the proteins VP1 having the amino acid sequence of SEQ ID NO: 14, VP2 having the amino acid sequence of SEQ ID NO: 15 and VP3 having the amino acid sequence of SEQ ID NO: 16, and the expression cassette includes a nucleotide sequence selected from the group comprising: SEQ ID NO: 3 or SEQ ID NO: 5.

### Pharmaceutical composition

In one aspect, the present invention relates to a pharmaceutical composition for delivering the FIX gene to target cells, which includes any of the above AAV5-based recombinant viruses in combination with one or more pharmaceutically acceptable excipients.

The active substance in the above composition is present in an effective amount, for example, in a biologically effective amount.

In particular embodiments, the present invention relates to a pharmaceutical composition comprising the AAV5-based recombinant virus of the invention in a pharmaceutically acceptable carrier or in other pharmaceutical agents, adjuvants, diluents, etc. For injection, the carrier will typically be a liquid carrier. For other methods of administration, the carrier may be either solid or liquid, such as sterile pyrogen-free water or sterile pyrogen-free phosphate-buffered saline solution. For inhalation administration, the carrier is respirable, and preferably is in a solid or liquid particulate form. As an injection medium, it is preferred to use water that contains the additives that are common for injection solutions, such as stabilizing agents, salts or saline, and/or buffers.

"Pharmaceutical composition" means a composition comprising the above AAV5-based recombinant virus of the invention and at least one of components selected from the group consisting of pharmaceutically acceptable and pharmacologically compatible excipients, such as fillers, solvents, diluents, carriers, auxiliary, distributing agents, delivery agents, preservatives, stabilizers, emulsifiers, suspending agents, thickeners, prolonged delivery controllers, the choice and proportions of which depend on the type and route of administration and dosage. Pharmaceutical compositions of the present invention and methods of preparation thereof will be undoubtedly apparent to those skilled in the art. The pharmaceutical compositions should preferably be manufactured in compliance with the GMP (Good Manufacturing Practice) requirements. The composition may comprise a buffer composition, tonicity agents, stabilizers and solubilizers.

"Pharmaceutically acceptable" means a material that does not have biological or other negative side effects, for example, the material can be administered to a subject without causing any undesirable biological effects. Thus, such pharmaceutical compositions may be used, for example, in transfection of a cell ex vivo or in in vivo administration of the AAV5-based recombinant virus of the invention directly to a subject.

The term "excipient" is used herein to describe any ingredient other than the above ingredients of the invention. These are substances of inorganic or organic nature which are used in the pharmaceutical production/manufacturing in order to give drug products the necessary physicochemical properties.

"Stabilizer" refers to an excipient or a mixture of two or more excipients that provide the physical and/or chemical stability of the active agent.

The term "buffer", "buffer composition", "buffering agent" refers to a solution, which is capable of resisting changes in pH by the action of its acid-base conjugate components, which allows the rAAV5 vector product to resist changes in pH. Generally, the pharmaceutical composition preferably has a pH in the range from 4.0 to 8.0. Examples of buffers used include, but are not limited to, acetate, phosphate, citrate, histidine, succinate, etc. buffer solutions.

The pharmaceutical composition is "stable" if the active agent retains physical stability and/or chemical stability and/or biological activity thereof during the specified shelf life at storage temperature, for example, of 2-8 °C. Preferably, the active agent retains both physical and chemical stability, as well as biological activity. Storage period is adjusted based on the results of stability test in accelerated or natural aging conditions.

A pharmaceutical composition according to the invention may be manufactured, packaged, or widely sold in the form of a single unit dose or a plurality of single unit doses in the form of a ready formulation. The term "single unit dose" as used herein refers to discrete quantity of a pharmaceutical composition containing a predetermined quantity of an active ingredient. The quantity of the active ingredient typically equals the dose of the active ingredient to be administered in a subject, or a convenient portion of such dose, for example, half or a third of such dose.

### Use

In one aspect, the present invention relates to the use of any of the above AAV5-based recombinant viruses or the above composition to deliver the FIX gene to target cells.

In one aspect, the present invention relates to the use of any of the above AAV5-based recombinant viruses or the above composition for providing the FIX protein to a subject that has hemophilia B and/or that does not have fully functional copies of the FIX gene.

In one aspect, the present invention relates to the use of any of the above AAV5-based recombinant viruses or the above composition for treating hemophilia B in a subject that has hemophilia B.

In one aspect, the present invention relates to a method for providing the FIX protein to a subject having hemophilia B, said method comprising administering a therapeutically effective amount of any of the above AAV5-based recombinant viruses or the above composition into the cells of the subject in need thereof.

In one aspect, the present invention relates to a method for delivering the FIX gene to the target cells of a subject having hemophilia B, said method comprising administering any of the above AAV5-based recombinant viruses or the above composition into the cells of the subject.

In one aspect, the present invention relates to a method for treating hemophilia B in a subject, said method comprising administering a therapeutically effective amount of any of the above AAV5-based recombinant viruses or the above composition into a subject that has hemophilia B.

Hemophilia B refers to an inherited coagulation disorder caused by deficiency or complete absence of coagulation factor IX (FIX). The prevalence of hemophilia B is about 1 in 40,000 newborn boys. The deficiency of coagulation factor is accompanied by spontaneous or induced hemorrhages in joints, muscles and internal organs.

The lack of fully functional copies of the FIX gene refers to inactivating mutations or deletions in all copies of the FIX gene in the genome, which result in the loss or defect of the function of the FIX gene.

Subject refers to any animal that is amenable to the techniques provided in the present description. In certain non-limiting embodiments, the subject is a human. Said subject may be either male or female, of any age.

A subject in need of delivering the FIX gene to target cells, or a subject in need of being provided with the FIX protein refers to a subject who has hemophilia B, or to a subject who has the deficiency of coagulation factor IX, or to a subject who has inactivating mutations or deletions in the FIX gene that lead to loss or defect in the function of the FIX gene.

Exemplary modes of administration include topical application, intranasal, inhalation, transmucosal, transdermal, enteral (e.g. oral, rectal), parenteral (e.g. intravenous, subcutaneous, intradermal, intramuscular) administrations, as well as direct tissue or organ injections.

Injectables can be prepared in conventional forms, either as liquid solutions or suspensions, solid forms suitable for the preparation of solution or suspensions in liquid prior to injection, or as emulsions. Alternatively, one may administer the above AAV5-based recombinant virus of the present invention in a local rather than systemic manner, for example in a depot or sustained-release formulation.

The AAV5-based recombinant virus is introduced into an organism in an effective amount. The AAV5-based recombinant virus is preferably introduced into an organism in a biologically effective amount. A "biologically effective" amount of the recombinant virus is an amount that is sufficient to cause transduction and expression of the nucleic acid sequence in the cell. If the virus is administered to a cell in vivo (e.g. the virus is administered to a subject, as described below), a "biologically-effective" amount of the viral vector is an amount that is sufficient to cause the transduction and expression of the nucleic acid sequence in the target cell.

Dosages of the above AAV5-based recombinant virus of the invention will depend on the mode of administration, the particular viral vector, and they can be determined in a routine manner. Exemplary doses for achieving therapeutic effects are viral titers of at least about 10⁵, 10⁶, 10⁷, 10⁸ 10⁹ 10¹⁰, 10¹¹, 10¹² 10¹³, 10¹⁴, 10¹⁵, 10¹⁶ transducing units or more, preferably about 10⁹ to 10¹⁵ transducing units, yet more preferably 10¹⁴ transducing units per kilogram.

The cell for administering the above AAV5-based recombinant virus of the invention may be a cell of any type, including but not limited to epithelial cells (e.g. skin, respiratory and gut epithelial cells), hepatic cells, muscle cells, pancreatic cells (including islet cells), hepatic cells, spleen cells, fibroblasts, endothelial cells, and the like.

The above AAV5-based recombinant virus of the invention is not used to modify the genetic integrity of human germ line cells.

### Examples

The following examples are provided for better understanding of the invention. These examples are for purposes of illustration only and are not to be construed as limiting the scope of the invention in any manner.

### Materials and general methods

### Recombinant DNA techniques

Standard methods were used to manipulate DNA as described in Sambrook, J. et al, Molecular cloning: A laboratory manual; Cold Spring Harbor Laboratory Press, Cold Spring Harbor, New York, 1989. The molecular biological reagents were used according to the manufacturer protocols. Briefly, plasmid DNA was produced for further manipulation in E. coli cells grown under selective antibiotic pressure so that the plasmids were not lost in the cell population. We isolated the plasmid DNA from cells using commercial kits, measured the concentration, and used it for cloning by restriction endonuclease treatment or PCR amplification. The DNA fragments were ligated to each other using ligases and transformed into bacterial cells for the selection of clones and further production. All resulting genetic constructs were confirmed by restriction patterns and complete Sanger sequencing.

### Gene synthesis

Desired gene segments were prepared from oligonucleotides made by chemical synthesis. Gene segments of 300 to 1000 bp long, which were flanked by unique restriction sites, were collected by renaturing oligonucleotides on top of each other, followed by PCR amplification from border primers. As a result, a mixture of fragments was produced, including the desired one. The fragments were cloned at restriction sites into intermediate vectors, following which the DNA sequences of the subcloned fragments were confirmed by DNA sequencing.

### DNA sequence determination

DNA sequences were determined by Sanger sequencing. DNA and protein sequences were analyzed and sequence data was processed in SnapGene Viewer 4.2 or higher for sequence creation, mapping, analysis, annotation and illustration.

### Culturing cell cultures

The experiments used HEK293 (Human Embryonic Kidney clone 293) and HUH7 (human hepatocellular carcinoma) cell lines. The suspended HEK293 cells used to produce AAV were cultured under standard conditions at 37° C and 5% CO₂ on a complete culture medium without FBS and antibiotic. The adherent HUH7 cells used to test the efficacy of AAV products were cultured under standard conditions at 37°C and 5% CO₂, on a complete DMEM medium supplemented with 10% FBS, antibiotic/antimycotic. The HUH7 cells were subcultured upon reaching 80-90% confluence. TrypLE Select enzyme (10x) was used to dissociate the cell monolayer. Cell viability was assessed using Trypan Blue stain and disposable cell counting chambers using an automatic Countess II counter.

### Assembly and purification of viral particles of AAV recombinant vectors

AAV virus particles comprising the codon-optimized variants of the FIX gene (hFIXco-v1 and hFIXco-v2) were assembled using HEK293 producing cells which were transfected with 3 plasmids as follows:
The plasmids pAAV-hFIXco-v1and pAAV-hFIXco-v2 comprising an AAV expression cassette for the expression of hFIXco-v1 and hFIXco-v2 transgenes, respectively (Fig. 1.);
A plasmid for expression of the AAV5 serotype Cap gene and the AAV2 serotype Rep gene. Each gene, using alternative reading frames, encodes several protein products;
A plasmid for expression of Ad5 (adenovirus serotype 5) genes that are required for assembly and packaging of AAV capsids.

After 72 hours, the cells were lysed and the viral particles were purified and concentrated using filtration, chromatography and ultracentrifugation methods. The titer of the viral particles was determined by quantitative PCR with primers and a sample that were specific for the region of the recombinant viral genome and expressed as the copy number of viral genomes per 1 ml.

### Transduction of cell cultures

The HUH7 cell line was pre-seeded into the wells of 12-well plates at a density of 10,000 cells/cm². After the cells were attached to the adhesive substrate, AAV preparations were introduced at MOI of 500,000 vg/cell. On day 7 following transduction, the content and activity of the FIX protein in the culture fluid were determined by ELISA, and the level of expression of the coagulation factor IX gene in cells was determined by reverse transcription-quantitative PCR, as described above. Studies involving the assessment of the level of the FIX protein in the culture fluid were performed in 6 independent experiments. Studies involving the assessment of the activity of the FIX protein being secreted and the level of coagulation factor IX gene expression in cells was performed in 3 independent experiments. Intact cells were used as a negative control.

### Determination of level of coagulation factor IX gene expression

The level of the *FIX* gene expression in cell cultures following transduction (or transfection) was determined by reverse transcription-quantitative PCR. Briefly, RNA was isolated from cellular sediments using the RNeasy Plus mini kit (Qiagen) according to the manufacturer's protocol. Reverse transcription was performed using GoScript reagent kit (Promega) according to the manufacturer's protocol; in particular, 500ng of RNA was initially sampled for the reverse transcription reaction, cDNA was produced using random and oligo dT primers; following the reverse transcription, the volume of cDNA was adjusted to 50 µl with sterile water. Quantitative PCR was performed using TaqMan technology using a qPCRmix-HS HighROX reaction mixture (Eurogene), according to the manufacturer's protocol, on a StepOne instrument (Applied Biosystems). We selected specific primers and probes for the target gene *(FIX)* and for the housekeeping gene *(GAPDH).* Standard curves were plotted using samples of plasmid DNAs (plDNAs) bearing the corresponding gene. For plotting each standard curve, 7 standard samples were prepared. A series of dilutions: from 20 million to 200 copies of plDNA per reaction (dilution factor was 1:10). For each test sample, the analysis was performed in three technical repeats and included doing RT minus control (to test for no DNA contamination in the RNA sample under study) and negative control. The results of determining the level of mRNA expression were presented in the following form: the target gene mRNA copy number in the sample normalized to *GAPDH* gene mRNA copy number.

### Determination of level of coagulation factor IX protein by ELISA

The content of the blood coagulation factor IX protein in the culture fluid following HUH7 transduction by target AAV5-FIX candidates, as well as that in the blood plasma of animals following the introduction of target viral products were assessed by sandwich method of non-competitive solid-phase enzyme immunoassay (ELISA) using a commercial kit. Briefly, samples of culture fluid and blood plasma diluted in a dilution buffer were introduced into 96-well plate wells sensitized with primary antibodies specific for coagulation factor IX. The same plate was loaded with standards for plotting a calibration curve, positive and negative controls. The plate was incubated for 2 hours at room temperature. The plate wells were washed with washing buffer prior to introducing biotinylated antibodies, solution of streptavidin peroxidase conjugate and TMB. A solution containing specific biotinylated detection antibodies to factor IX was introduced, and the plate was incubated for 1 hour at room temperature. Streptavidin peroxidase conjugate solution was then added to the resulting complex, and the plate was incubated for 30 minutes at room temperature. TMB solution was introduced to visualize the enzyme reaction. Upon achieving the required degree of staining intensity, a stop solution was added to all wells to stop the reaction. The optical density of the solutions in the plate wells was then measured. The concentration of coagulation factor IX in the test samples was determined by the calibration curve considering the preliminary dilution of the samples.

### Determination of activity of coagulation factor IX protein

Coagulation factor IX is vitamin K-dependent, and the synthesis of the active form of factor IX by cell culture needs the presence of vitamin K in the growth medium. In this connection, during transduction of HUH7, vitamin K1 at a concentration of 500 ng/ml was added to the composition of the complete growth medium.

The activity of the coagulation factor IX protein in the culture fluid following transduction of HUH7 with target products was assessed by chromogenic method using a commercial kit.

Briefly, culture fluid samples, standards and control solutions diluted in Tris-BSA reaction buffer were introduced into 96-well plate wells. Reagent 1 (FX-FVIII) was then added thereto. Following 2-minute incubation at a temperature of 37 ⁰ C, reagent 2 (activating agent) was introduced. The plate was incubated for 3 minutes at a temperature of 37 ⁰ C. Reagent 3 (chromogenic substrate) was then introduced into plate wells, and the plate was incubated for 2 minutes. 20% acetic acid solution was then added to stop the reaction. The resulting FXa hydrolyzes the chromogenic substrate, thus resulting in the release of paranitroaniline, the amount of which (detected by optical density) is directly proportional to the concentration of factor IX (FIX) in the sample.

### In vivo study on laboratory animals

The experiments were performed on C57BL/6 mice (males aged 6-8 weeks). The products were administered to animals by way of a single intravenous injection into the tail vein. A buffer solution containing no AAV was adminitered into the negative control group of animals. Blood plasma was collected on the day of injection prior to administration of the products, and then on days 7, 14, 21, 28, 35 and 42 following the administration of the products.

### Example 1. Modification of FIX gene sequence using codon optimization algorithm

The product that we developed is a suspension of modified recombinant capsids of adenoassociated virus serotype 5 (AAV5) bearing an expression cassette encoding the human coagulation factor IX (hFIX) gene under the control of the liver tissue-specific transthyretin (TTR) promoter.

The wild-type nucleic acid employed was a nucleic acid that encodes the wild-type human coagulation factor IX with the naturally-occurring R338L mutation (known as Padua mutation), and includes the nucleotide sequence of SEQ ID NO: 17. The given wild-type nucleic acid is used as a control.

Further, to increase the efficiency of expression, the natural sequence of the coagulation factor IX gene was modified using a codon optimization algorithm.

Codon optimization of the wild-type nucleic acid of coagulation factor IX having the nucleotide sequence of SEQ ID NO: 17 resulted in producing a number of codon-optimized nucleic acids which were further tested for the level of expression and activity of the coagulation factor IX protein, as compared to those of control (wild-type nucleic acid having SEQ ID NO: 17), within the AAV5-based product.

All codon-optimized nucleic acids showed increased level of production of the coagulation factor IX protein as compared to that of wild type; further, most of the codon-optimized nucleic acids showed a slightly increased level of production of the coagulation factor IX protein as compared to that of wild type, and only two codon-optimized variants of nucleic acid according to the invention having the nucleotide sequence of SEQ ID NO: 2 (hFIXco-v1) and SEQ ID NO: 4 (hFIXco-v2) surprisingly showed the best results, in particular, increased level of expression of the coagulation factor IX gene and several fold increased level of production of the coagulation factor IX protein, which fact, in turn, resulted in the increased activity of the AAV5-based product which comprised SEQ ID NO: 2 (hFIXco-v1) or SEQ ID NO: 4 (hFIXco-v2) (see Examples 2, 3 and 4).

Codon-optimized nucleic acids having the nucleotide sequences of SEQ ID NO: 2 and SEQ ID NO: 4 have an increased codon adaptation index (a standard measure for evaluating a sequence for codon usage frequencies) for mammalian cells as compared to that of the wild-type nucleic acid having the nucleotide sequence of SEQ ID NO: 17.

Codon-optimized nucleic acids having the nucleotide sequences of SEQ ID NO: 2 and SEQ ID NO: 4 are referred to in the examples below as hFIXco-v1 and hFIXco-v2, respectively.

### Example 2. Assembly of a genetic construct comprising an AAV expression cassette having variants of the recombinant codon-optimized coagulation factor IX gene (hFIXco-v1 and hFIXco-v2).

The target plasmids pAAV-hFIXco-v1or pAAV-hFIXco-v2 (Fig. 1.) intended for producing the AAV5 viral vectors with an expression cassette comprising a codon-optimized variant of the coagulation factor IX gene (SEQ ID NO: 2 or SEQ ID NO: 4) were produced by successively replacing the sequence of the modified green fluorescent protein and the CMV promoter in the original construct pAAV-GFP using the restriction enzyme ligase method of cloning, with the codon-optimized sequence of the coagulation factor IX gene and TTR promoter, respectively, which were synthesized *de novo* from oligonucleotides generated by chemical synthesis.

The final vector contains all the necessary elements for expression and assembly of the gene in the recombinant AAV genome:
1) ITRs at the ends of the sequence that is encapsidated into a viral capsid;
2) elements for expression of the target gene (promoter, enhancer, intron, Kozak sequence, transgene, polyadenylation site);
3) bacterial replication origin and antibiotic resistance gene to produce plasmid DNA in bacterial cells.

### Example 3. Creation of viral products expressing coagulation factor IX

The target plasmids pAAV-hFIXco-v1 and pAAV-hFIXco-v2 (Fig. 1) along with the other plasmids necessary for producing the recombinant AAV viral particles (see above) were used for producing the AAV5-hFIXco-v1 and AAV5-hFIXco-v2 products bearing the codon-optimized versions of the coagulation factor IX gene (*hFIXco-and hFIXco v1-v2,* respectively). The bioprocessing resulted in recombinant AAV5-hFIXco-v1 and AAV5-hFIXco-v2 viral particles comprising expression cassettes with codon-optimized variants of the coagulation factor IX gene *(hFIXco-v1 and hFIXco-v2).* The purified AAV5-hFIXco-v1 and AAV5-hFIXco-v2 products used for *in vitro* and *in vivo* studies were prepared using standard buffers and excipients that are safe and do not alter the AAV properties. The AAV5-hFIX-wt product comprising an expression cassette with the naturally occurring coagulation factor IX gene (the wild-type gene including the naturally-occurring R338L mutation, see Example 1) was further produced using the above technology to serve as a reference product.

### Example 4. Testing of in vitro performance of AAV5-hFIXco-v1 and AAV5-hFIXco-v2 products

Prior to animal testing, the purified AAV5-hFIXco-v1 and AAV5-hFIXco-v2 products were tested in *vitro.* The experiments were performed using the HUH7 adherent cell line (Fig. 2, 3 and 4). The cell line's cells were plated into the wells of 12-well plates at a density of 10,000 cells/cm². After the cells were attached to the adhesive substrate, AAV preparations were introduced at MOI of 500,000 vg/cell. On day 7 following transduction, the content and activity of the FIX protein in the culture fluid were determined by ELISA, and the level of expression of the coagulation factor IX gene in cells was determined by reverse transcription-quantitative PCR, as described above. All samples were run in triplicates. Intact cells were used as a negative control.

It has been shown that the AAV5-hFIXco-v1 and AAV5-hFIXco-v2 products that we developed, which bear codon-optimized versions of the coagulation factor IX gene (*hFIXco-v1 and hFIXco-v2),* provide for efficient delivery of the coagulation factor IX transgene into cells and ensure the production of the target protein, which facts are confirmed by the data of quantitative real-time PCR, ELISA and analysis of the activity of the blood coagulation factor IX protein (Fig. 2, 3 and 4). In this connection, when using the AAV5-hFIXco-v1 and AAV5-hFIXco-v2 products comprising a codon-optimized sequence of the coagulation factor IX gene, the level of expression of the coagulation factor IX gene is 1.8 times greater for the AAV5-hFIXco-v1 product and 2.8 times greater for the AAV5-hFIXco-v2 product as compared to using a product having the naturally occurring version of the coagulation factor IX gene (AAV5-hFIX-wt) (Fig. 2). Furthermore, when using the AAV5-hFIXco-v1 and AAV5-hFIXco-v2 products comprising a codon-optimized sequence of the coagulation factor IX gene, the level of production of the coagulation factor IX protein is 1.6 times greater for the AAV5-hFIXco-v1 product and 2.1 times greater for the AAV5-hFIXco-v2 product as compared to using a product having the naturally occurring version of the coagulation factor IX gene (AAV5-hFIX-wt) (Fig. 3). It should also be noted that, when using the AAV5-hFIXco-v1 and AAV5-hFIXco-v2 products comprising a codon-optimized sequence of the coagulation factor IX gene, the level of activity of the coagulation factor IX protein is 2.1 times greater for the AAV5-hFIXco-v1 product and 2.9 times greater for the AAV5-hFIXco-v2 product as compared to using a product having the naturally occurring version of the coagulation factor IX gene (AAV5-hFIX-wt) (Fig. 4).

### Example 5. Testing of in vivo performance of AAV5-hFIXco-v1 and AAVS-hFIXco-v2 products

*In vivo* studies of the AAV5-hFIXco-v1 and AAV5-hFIXco-v2 products used C57BL/6 line laboratory mice. The AAV product dose used in the study was 4x10¹¹ VG/mouse. A control solution without AAV was used as a negative control. The products were administered to animals by way of a single intravenous injection into the tail vein. Blood plasma was collected on the day of injection prior to administration of the products, and then on days 7, 14, 21, 28, 35 and 42 following the administration of the products. The level of the coagulation factor IX protein in the blood plasma samples was determined by ELISA, as described above.

The *in vivo* studies have shown that the use of the AAV5-hFIXco-v1 product comprising a codon-optimized sequence of the coagulation factor IX gene hFIXco-v1 shows a significantly greater level of coagulation factor IX protein in the animals' blood (2.2 to 2.3 times greater) on days 21 and 28 following product administration as compared to the use of the product having the naturally-occurring version of the coagulation factor IX gene (AAV5-hFIX-wt) (Fig 4A). With the use of the AAV5-hFIXco-v2 product comprising a codon-optimized sequence of the coagulation factor IX gene hFIXco-v2, there is observed a significantly greater level of the coagulation factor IX protein in the animals' blood (1.8 to 2.5 times greater) on days 14, 21, 28, 35 and 42 following product administration as compared to the use of the product having the naturally-occurring version of the coagulation factor FIX gene (AAV5-hFIX-wt) (Fig 5).

Thus, the AAV5-based recombinant viruses that we developed, which bear codon-optimized versions of the coagulation factor IX gene (AAV5-hFIXco-v1 or AAV5-hFIXco-v2), have an advantage over the AAV5 vector having a naturally-occurring version of the coagulation factor IX gene and have potential for gene therapy of Hemophilia B.

## Claims

1. An isolated codon-optimized nucleic acid encoding the FIX (coagulation factor IX) protein having the amino acid sequence of SEQ ID NO: 1, that includes a nucleotide sequence that is selected from the group comprising: SEQ ID NO: 2 or SEQ ID NO: 4.

2. An expression cassette that includes the codon-optimized nucleic acid according to claim 1.

3. The expression cassette according to claim 2, comprising the following elements in the 5'-end to 3'-end direction:
a left-hand (first) ITR (inverted terminal repeats);
TTR promoter (transthyretin promoter);
an intron of the hBG1 gene (an intron-bearing fragment of the human β-globin gene);
the codon-optimized nucleic acid according to claim 1;
an hGH1 polyadenylation signal (human growth hormone gene polyadenylation signal);
a right-hand (second) ITR.

4. The expression cassette according to claim 3, that includes a nucleotide sequence that is selected from the group comprising: SEQ ID NO: 3 or SEQ ID NO: 5.

5. An expression vector that includes the codon-optimized nucleic acid according to claim 1 or the expression cassette according to any of claims 2 to 4.

6. An isolated AAV5 (adeno-associated virus serotype 5)-based recombinant virus for increasing the FIX gene expression in target cells, which includes the codon-optimized nucleic acid according to claim 1 or the expression cassette according to any of claims 2 to 4.

7. The AAV5-based recombinant virus according to claim 6, wherein the capsid includes the AAV5 protein VP1, preferably
(i) wherein the capsid includes the AAV5 protein VP1 having the amino acid sequence of SEQ ID NO: 11 or
(ii) wherein the capsid includes the AAV5 protein VP1 having the amino acid sequence of SEQ ID NO: 11 with one or more point mutations, preferably wherein the capsid includes the AAV5 protein VP1 having the amino acid sequence of SEQ ID NO: 14.

8. The AAV5-based recombinant virus according to any of Claims 6 or 7, wherein the capsid includes the AAV5 protein VP1 having the amino acid sequence of SEQ ID NO: 11 or the amino acid sequence of SEQ ID NO: 11 with one or more point mutations, and the expression cassette includes the following elements in the 5'-end to 3'-end direction:
a left-hand (first) ITR (inverted terminal repeats);
a TTR promoter;
an intron of the hBG1 gene (an intron-bearing fragment of the human β-globin gene);
the codon-optimized nucleic acid according to claim 1;
an hGH1 polyadenylation signal (human growth hormone gene polyadenylation signal);
a right-hand (second) ITR.

9. The AAV5-based recombinant virus according to claim 8, wherein the capsid includes the AAV5 protein VP1 having the amino acid sequence of SEQ ID NO: 11 or the amino acid sequence of SEQ ID NO: 11 with one or more point mutations, and the expression cassette comprises a nucleotide sequence selected from the group comprising SEQ ID NO: 3 or SEQ ID NO: 5.

10. The AAV5-based recombinant virus according to claim 8 or 9, wherein the AAV5 protein VP1 having the amino acid sequence of SEQ ID NO: 11 with one or more point mutations is the amino acid sequence of SEQ ID NO: 14.

11. A pharmaceutical composition for delivering the FIX gene to target cells, comprising the AAV5-based recombinant virus according to any of claims 6 to 10 in combination with one or more pharmaceutically acceptable excipients.

12. In vitro use of the AAV5-based recombinant virus according to claims 6 to 10 or the composition according to claim 11 for delivering the FIX gene to target cells.

13. AAV5-based recombinant virus according to any of claims 6 to 10 or the composition according to claim 11 for use in the treatment of a subject that has hemophilia B and/or that does not have fully functional copies of the FIX gene, wherein the AAV5-based recombinant virus provides the FIX protein to the subject.

14. AAV5-based recombinant virus according to any of claims 6 to 10 or the composition according to claim 11 for use in the treatment of hemophilia B in a subject that has hemophilia B.

## Patentansprüche

1. Isolierte codonoptimierte Nukleinsäure, codierend für das FIX-Protein (Gerinnungsfaktor IX-Protein) mit der Aminosäuresequenz von SEQ ID NO: 1, die eine Nukleotidsequenz einschließt, die aus der Gruppe ausgewählt ist, umfassend: SEQ ID NO: 2 oder SEQ ID NO: 4.

2. Expressionskassette, einschließend die codonoptimierte Nukleinsäure nach Anspruch 1.

3. Expressionskassette nach Anspruch 2, umfassend die folgenden Elemente in der Richtung vom 5'-Ende zum 3'-Ende:
ein linksseitiges (erstes) ITR (Inverted Terminal Repeat);
einen TTR-Promotor (Transthyretin-Promoter);
ein Intron des hBG1-Gens (ein Intron-tragendes Fragment des humanen β-Globin-Gens);
die codonoptimierte Nukleinsäure nach Anspruch 1;
ein hGH1-Polyadenylierungssignal (Polyadenylierungssignal des humanen Wachstumshormon-Gens);
ein rechtes (zweites) ITR.

4. Expressionskassette nach Anspruch 3, einschließend eine Nukleotidsequenz, die aus der Gruppe ausgewählt ist, umfassend: SEQ ID NO: 3 oder SEQ ID NO: 5.

5. Expressionsvektor, einschließend die codonoptimierte Nukleinsäure nach Anspruch 1 oder die Expressionskassette nach einem der Ansprüche 2 bis 4.

6. Isoliertes rekombinantes Virus auf Basis des AAV5 (Adeno-assoziiertes Virus Serotyp 5) zum Erhöhen der FIX-Genexpression in Zielzellen, das die codonoptimierte Nukleinsäure nach Anspruch 1 oder die Expressionskassette nach einem der Ansprüche 2 bis 4 einschließt.

7. Rekombinantes Virus auf Basis des AAV5 nach Anspruch 6, wobei das Kapsid das AAV5-Protein VP1 einschließt, vorzugsweise
(i) wobei das Kapsid das AAV5-Protein VP1 mit der Aminosäuresequenz von SEQ ID NO: 11 einschließt, oder
(ii)wobei das Kapsid das AAV5-Protein VP1 mit der Aminosäuresequenz von SEQ ID NO: 11 mit einer oder mehreren Punktmutationen einschließt, vorzugsweise wobei das Kapsid das AAV5-Protein VP1 mit der Aminosäuresequenz von SEQ ID NO: 14 einschließt.

8. Rekombinantes Virus auf Basis des AAV5 nach einem der Ansprüche 6 oder 7, wobei das Kapsid das AAV5-Protein VP1 mit der Aminosäuresequenz von SEQ ID NO: 11 oder der Aminosäuresequenz von SEQ ID NO: 11 mit einer oder mehreren Punktmutationen einschließt, und die Expressionskassette die folgenden Elemente in der Richtung von 5'-Ende zu 3'-Ende einschließt:
ein linksseitiges (erstes) ITR (Inverted Terminal Repeat);
einen TTR-Promotor;
ein Intron des hBG1-Gens (ein Intron-tragendes Fragment des humanen β-Globin-Gens);
die codonoptimierte Nukleinsäure nach Anspruch 1;
ein hGH1-Polyadenylierungssignal (Polyadenylierungssignal des humanen Wachstumshormon-Gens);
ein rechtes (zweites) ITR.

9. Rekombinantes Virus auf Basis des AAV5 nach Anspruch 8, wobei das Kapsid das AAV5-Protein VP1 mit der Aminosäuresequenz von SEQ ID NO: 11 oder der Aminosäuresequenz von SEQ ID NO: 11 mit einer oder mehreren Punktmutationen einschließt, und die Expressionskassette eine Nukleotidsequenz umfasst, die aus der Gruppe ausgewählt ist, die SEQ ID NO: 3 oder SEQ ID NO: 5 umfasst.

10. Rekombinantes Virus auf Basis des AAV5 nach Anspruch 8 oder 9, wobei das AAV5-Protein VP1 mit der Aminosäuresequenz von SEQ ID NO: 11 mit einer oder mehreren Punktmutationen die Aminosäuresequenz von SEQ ID NO: 14 ist.

11. Pharmazeutische Zusammensetzung zur Abgabe des FIX-Gens an Zielzellen, umfassend das rekombinante Virus auf Basis des AAV5 nach einem der Ansprüche 6 bis 10 in Kombination mit einem oder mehreren pharmazeutisch unbedenklichen Hilfsstoffen.

12. In-vitro-Verwendung des rekombinanten Virus auf Basis des AAV5 nach den Ansprüchen 6 bis 10 oder der Zusammensetzung nach Anspruch 11 zur Abgabe des FIX-Gens an Zielzellen.

13. Rekombinantes Virus auf Basis des AAV5 nach einem der Ansprüche 6 bis 10 oder Zusammensetzung nach Anspruch 11 zur Verwendung bei der Behandlung eines Subjekts, das an Hämophilie B leidet und/oder keine voll funktionsfähigen Kopien des FIX-Gens besitzt, wobei das rekombinante Virus auf Basis des AAV5 dem Subjekt das FIX-Protein bereitstellt.

14. Rekombinantes Virus auf Basis des AAV5 nach einem der Ansprüche 6 bis 10 oder Zusammensetzung nach Anspruch 11 zur Verwendung bei der Behandlung von Hämophilie B bei einem Subjekt, das an Hämophilie B leidet.

## Revendications

1. Acide nucléique isolé à codons optimisés codant pour la protéine FIX (facteur IX de coagulation) ayant la séquence d'acides aminés de SEQ ID NO : 1, qui inclut une séquence de nucléotide qui est sélectionnée dans le groupe comprenant : SEQ ID NO : 2 ou SEQ ID NO : 4.

2. Cassette d'expression qui inclut l'acide nucléique à codons optimisés selon la revendication 1.

3. Cassette d'expression selon la revendication 2, comprenant les éléments suivants dans la direction de l'extrémité 5' à l'extrémité 3' :
une (première) ITR (répétitions terminales inversées) gauche ;
un promoteur de TTR (promoteur de transthyrétine) ;
un intron du gène hBG1 (un fragment porteur d'intron du gène humain β-globine);
l'acide nucléique à codons optimisés selon la revendication 1 ;
un signal de polyadénylation d'hGH1 (signal de polyadénylation du gène d'hormone de croissance humaine) ;
un (second) ITR droit.

4. Cassette d'expression selon la revendication 3, qui inclut une séquence de nucléotide qui est sélectionnée dans le groupe comprenant : SEQ ID NO : 3 ou SEQ ID NO : 5.

5. Vecteur d'expression qui inclut l'acide nucléique à codons optimisés selon la revendication 1 ou la cassette d'expression selon l'une quelconque des revendications 2 à 4.

6. Virus recombinant isolé basé sur l'AAV5 (virus adéno-associé de sérotype 5) pour augmenter l'expression du gène FIX dans les cellules cibles, qui inclut l'acide nucléique à codons optimisés selon la revendication 1 ou la cassette d'expression selon l'une quelconque des revendications 2 à 4.

7. Virus recombinant basé sur l'AAV5 selon la revendication 6, dans lequel la capside inclut la protéine VP1 de l'AAV5, de préférence
(i) dans lequel la capside inclut la protéine VP1 de l'AAV5 ayant la séquence d'acides aminés de SEQ ID NO : 11 ou
(ii) dans lequel la capside inclut la protéine VP1 de l'AAV5 ayant la séquence d'acides aminés de SEQ ID NO : 11 avec une ou plusieurs mutations ponctuelles, de préférence dans lequel la capside inclut la protéine VP1 de l'AAV5 ayant la séquence d'acides aminés de SEQ ID NO : 14.

8. Virus recombinant basé sur l'AAV5 selon l'une quelconque des revendications 6 ou 7, dans lequel la capside inclut la protéine VP1 de l'AAV5 ayant la séquence d'acides aminés de SEQ ID NO : 11 ou la séquence d'acides aminés de SEQ ID NO : 11 avec une ou plusieurs mutations ponctuelles, et la cassette d'expression inclut les éléments suivants dans la direction de l'extrémité 5' à l'extrémité 3' :
une (première) ITR (répétitions terminales inversées) gauche ;
un promoteur de TTR ;
un intron du gène hBG1 (un fragment porteur d'intron du gène humain β-globine) ;
l'acide nucléique à codons optimisés selon la revendication 1 ;
un signal de polyadénylation d'hGH1 (signal de polyadénylation du gène d'hormone de croissance humaine) ;
un (second) ITR droit.

9. Virus recombinant basé sur l'AAV5 selon la revendication 8, dans lequel la capside inclut la protéine VP1 de l'AAV5 ayant la séquence d'acides aminés de SEQ ID NO : 11 ou la séquence d'acides aminés de SEQ ID NO : 11 avec une ou plusieurs mutations ponctuelles, et la cassette d'expression comprend une séquence de nucléotide sélectionnée dans le groupe comprenant la SEQ ID NO : 3 ou SEQ ID NO : 5.

10. Virus recombinant basé sur l'AAV5 selon la revendication 8 ou 9, dans lequel la protéine VP1 de l'AAV5 ayant la séquence d'acides aminés de SEQ ID NO : 11 avec une ou plusieurs mutations ponctuelles est la séquence d'acides aminés de SEQ ID NO : 14.

11. Composition pharmaceutique pour délivrer le gène FIX aux cellules cibles, comprenant le virus recombinant basé sur l'AAV5 selon l'une quelconque des revendications 6 à 10 en combinaison avec un ou plusieurs excipients pharmaceutiquement acceptables.

12. Utilisation in vitro du virus recombinant basé sur l'AAV5 selon les revendications 6 à 10 ou de la composition selon la revendication 11 pour délivrer le gène FIX aux cellules cibles.

13. Virus recombinant basé sur l'AAV5 selon l'une quelconque des revendications 6 à 10 ou composition selon la revendication 11 pour une utilisation dans le traitement d'un sujet qui a une hémophilie B et/ou qui n'a pas de copies entièrement fonctionnelles du gène FIX, dans lequel le virus recombinant basé sur l'AAV5 fournit la protéine FIX au sujet.

14. Virus recombinant basé sur l'AAV5 selon l'une quelconque des revendications 6 à 10 ou composition selon la revendication 11 pour une utilisation dans le traitement de l'hémophilie B chez un sujet qui a une hémophilie B.
